# EUROPÄISCHE PATENTSCHRIFT

(11) **EP 1 697 309 B1**
(45) Veröffentlichungstag und Bekanntmachung des Hinweises auf die Patenterteilung: **23.04.2014**
(21) Anmeldenummer: 04803999.4
(22) Anmeldetag: 17.12.2004
(51) Int. Cl.: C07C 237/42, C07C 271/10, C07C 271/26, C07C 275/24, C07C 275/28, C07C 309/66, C07C 311/06, C07C 323/60, C07C 327/34, C07C 333/04, C07C 333/08, A01N 37/18, A01P 13/00

(54) **BENZOYLSUBSTITUIERTE PHENYLALANIN-AMIDE**
BENZOYL-SUBSTITUTED PHENYLALANINE AMIDES
AMIDES DE PHENYLALANINE A SUBSTITUTION BENZOYLE

(30) Priorität: 19.12.2003 DE 10360395
(43) Veröffentlichungstag der Anmeldung: 06.09.2006
(73) Patentinhaber: BASF SE, 67056 Ludwigshafen (DE)
(72) Erfinder: WITSCHEL, Matthias, 67098 Bad Dürkheim (DE); PUHL, Michael, 68623 Lampertheim (DE); HAMPRECHT, Gerhard, 69469 Weinheim (DE); PARRA RAPADO, Liliana, 77654 Offenburg (DE); MIßLITZ, Ulf, 67433 Neustadt (DE); ZAGAR, Cyrill, 68167 Mannheim (DE); PLATH, Peter, 67227 Frankenthal (DE); REINHARD, Robert, 67065 Ludwigshafen (DE); SIEVERNICH, Bernd, 67454 Hassloch (DE); LIEBL, Rex, 67146 Deidesheim (DE)
(86) Internationale Anmeldenummer: PCT/EP2004/014392
(87) Internationale Veröffentlichungsnummer: WO 2005/061443

(56) Entgegenhaltungen:
- WO-A-97/05865
- WO-A-03/066576
- GB-A- 2 369 117
- US-A- 4 004 008
- DATABASE CA [Online] CHEMICAL ABSTRACTS SERVICE, COLUMBUS, OHIO, US; SOLOSHONOK, VADIM A. ET AL: "Gold(I)-catalyzed asymmetric aldol reactions of fluorinated benzaldehydes with an .alpha.-isocyanoacetamide" XP002329711 gefunden im STN Database accession no. 1994:534014 & TETRAHEDRON: ASYMMETRY , 5(6), 1091-4 CODEN: TASYE3; ISSN: 0957-4166, 1994,
- DATABASE CA [Online] CHEMICAL ABSTRACTS SERVICE, COLUMBUS, OHIO, US; SOLOSHONOK, VADIM A. ET AL: "Gold(I)-catalyzed asymmetric aldol reaction of methyl isocyanoacetate with fluorinated benzaldehydes" XP002329712 gefunden im STN Database accession no. 1995:11127 & TETRAHEDRON LETTERS , 35(17), 2713-16 CODEN: TELEAY; ISSN: 0040-4039, 1994,
- DATABASE CA [Online] CHEMICAL ABSTRACTS SERVICE, COLUMBUS, OHIO, US; JOMMI, GIANCARLO ET AL: "Asymmetric synthesis of .beta.-hydroxy-.alpha.-amino acids by condensation of aliphatic and aromatic aldehydes with zinc(II) and copper(II) complexes of (1R)-3-hydroxymethylene bornan-2-one glycine imines" XP002329713 gefunden im STN Database accession no. 1995:177010 & GAZZETTA CHIMICA ITALIANA , 124(7), 299-300 CODEN: GCITA9; ISSN: 0016-5603, 1994,
- DATABASE CA [Online] CHEMICAL ABSTRACTS SERVICE, COLUMBUS, OHIO, US; SOLOSHONOK, VADIM A. ET AL: "Transition Metal/Base-Catalyzed Aldol Reactions of Isocyanoacetic Acid Derivatives with Prochiral Ketones, a Straightforward Approach to Stereochemically Defined .beta.,.beta.-Disubstituted-.beta.-hydroxy - .alpha.-amino Acids.1 Scope and Limitations" XP002329714 gefunden im STN Database accession no. 1997:315104 & JOURNAL OF ORGANIC CHEMISTRY , 62(11), 3470-3479 CODEN: JOCEAH; ISSN: 0022-3263, 1997,
- PATENT ABSTRACTS OF JAPAN Bd. 016, Nr. 128 (C-0924), 2. April 1992 (1992-04-02) & JP 03 294253 A (SHIONOGI & CO LTD), 25. Dezember 1991 (1991-12-25) in der Anmeldung erwähnt
- PATENT ABSTRACTS OF JAPAN Bd. 1999, Nr. 02, 26. Februar 1999 (1999-02-26) & JP 10 298151 A (JAPAN ENERGY CORP), 10. November 1998 (1998-11-10) in der Anmeldung erwähnt

## Beschreibung

Die vorliegende Erfindung betrifft benzoylsubstituierte Phenylalanin-Amide der Formel I 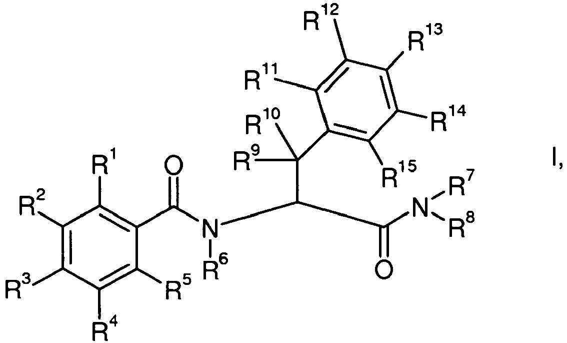 in der die Variablen die folgenden Bedeutungen haben:
- R¹: Halogen, Cyano, C₁-C₆-Alkyl, C₁-C₆-Halogenalkyl, C₁-C₆-Halogenalkoxy, Nitro, Hydroxycarbonyl, C₁-C₆-Alkoxycarbonyl, C₁-C₆-Halogenalkylthio oder Phenyl;
- R², R³, R⁴, R⁵: Wasserstoff, Halogen, Cyano, C₁-C₆-Alkyl, C₁-C₆-Halogenalkyl, C₁-C₆-Alkoxy, C₁-C₆-Halogenalkoxy, Nitro, Amino, C₁-C₆-Alkylamino, Di(C₁-C₆-alkyl)amino, C₁-C₆-Alkylthio oder C₁-C₆-Alkoxycarbonyl;
- R⁶, R⁷: Wasserstoff, Hydroxy oder C₁-C₆-Alkoxy;
- R⁸: C₁-C₆-Alkyl, C₁-C₄-Cyanoalkyl oder C₁-C₆-Halogenalkyl;
- R⁹: OR¹⁶
- R¹⁰: Wasserstoff oder C₁-C₆-Alkyl;
- R¹¹, R¹²: Wasserstoff, Halogen, Cyano, C₁-C₆-Alkyl, C₁-C₆-Halogenalkyl, Hydroxy, C₁-C₆-Alkoxy, C₁-C₆-Halogenalkoxy, Hydroxy, Nitro, Hydroxy-C₁-C₄-alkyl, C₁-C₆-Alkoxy-C₁-C₄-alkyl, Tri(C₁-C₆-alkyl)silyloxy-C₁-C₄-alkyl, C₁-C₄-Alkylthio, (Hydroxycarbonyl)C₁-C₆-alkyl, (C₁-C₆-Alkoxycarbonyl)-C₁-C₆-alkyl, (Hydroxycarbonyl)-C₂-C₆-alkenyl, (C₁-C₆-Alkoxycarbonyl)-C₂-C₆-alkenyl, (Hydroxycarbonyl)-C₁-C₄-alkoxy, (C4₁-C₄-Alkoxycarbonyl)-C₁-C₄-alkoxy, (C₁-C₄-Alkylcarbonyl)oxy-C₁-C₄-alkyl, Hydroxycarbonyl-C₁-C₄-alkoxy-C₁-C₄-alkyl, (C₁-C₄-Alkylsulfonyl)oxy-C₁-C₄-alkyl, C₁-C₄-Alkyl-O-C(O)-[C₁-C₄-alkyl-O]₃-C₁-C₄-alkyl, Carbamoyloxy-C₁-C₄-alkyl, (C₁-C₄-Alkylaminocarbonyl)oxy-C₁-C₄-alkyl, [Di(C₁-C₄-alkyl)aminocarbonyl]oxy-C₁-C₄-alkyl, [(C₁-C₄-Halogenalkylsulfonyl)-aminocarbonyl]oxy-C₁-C₄-alkyl, Benzyloxy, wobei der Phenylring durch 1 bis 3 Reste aus der Gruppe Halogen und C₁-C₄-Alkyl substituiert sein kann,
Amino, C₁-C₄-Alkylamino, Di(C₁-C₄-alkyl)amino, C₁-C₄-Alkylsulfonyl-amino, C₁-C₄-Halogenalkylsulfonylamino, C₁-C₄-Alkylcarbonylamino, Carbamoylamino, (C₁-C₄-Alkylamino)-carbonylamino, [Di(C₁-C₄-alkyl)-amino]carbonylamino, [(C₁-C₄-Halogenalkylsulfonyl)aminocarbonyl]-amino, Phenyl oder Heterocyclyl, wobei der Phenyl- und der Heterocyclylrest der zwei letztgenannten Substituenten ein bis drei Reste aus folgender Gruppe tragen kann: Halogen, Nitro, C₁-C₄-Alkyl, C₁-C₄-Halogenalkyl, Hydroxycarbonyl und C₁-C₆-Alkoxycarbonyl ;
- R¹³, R¹⁴, R¹⁵: Wasserstoff, Halogen, Cyano, C₁-C₆-Alkyl, C₁-C₆-Halogenalkyl, C₁-C₆-Alkoxy, C₁-C₆-Halogenalkoxy, Hydroxy, Nitro, C₁-C₆-Alkylthio oder Benzyloxy;
- R¹⁶,: Wasserstoff, C₁-C₆-Alkyl, Tri(C₁-C₆-alkyl)silyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, C₁-C₆-Halogenalkylsulfonylamino-carbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N-(Di-C₁-C₆-alkylamino)-imino-C₁-C₆-alkyl,
wobei die genannten Alkyl-, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl oder C₁-C₄-Alkylcarbonyloxy;
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl oder Heterocyclyl-C₁-C₆-alkylcarbonyl,
wobei der Phenyl- und der Heterocyclyl-Rest der 17 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
SQ₂R¹⁷;
-C(O)-[C₁-C₄-alkyl-O]₃-C₁-C₄-Alkyl; oder -C(O)-O-C₁-C₄-Alkyl-O-Phenyl, wobei der Phenylrest gegebenenfalls durch ein bis drei Reste aus der Gruppe Halogen und C₁-C₄-Alkyl substituiert sein kann;
- R¹⁷: C₁-C₆-Alkyl, C₁-C₆-Halogenalkyl oder Phenyl,
wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

Außerdem betrifft die Erfindung Verfahren und Zwischenprodukte zur Herstellung von Verbindungen der Formel 1, Mittel welche diese enthalten sowie die Verwendung dieser Derivate oder diese enthaltende Mittel zur Schadpflanzenbekämpfung.

Aus der Literatur, beispielsweise aus WO 03/066576, sind herbizid wirksame Phenylalaninderivate, welche in β-Position unsubstituiert sind oder ggf. durch Halogen substituierte Alkyl-, Alkenyl- oder Alkinylreste tragen, bekannt.

Benzoylsubstituierte Aminosäureamide mit pharmazeutischer Wirksamkeit werden u.a. in WO 97/05865, GB 2369117, JP 10/298151 und JP 03/294253 beschrieben.

Die herbiziden Eigenschaften der bisher bekannten Verbindungen bzw. die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die benzoylsubstituierten Phenylalanin-Amide der Formel I sowie deren herbizide Wirkung gefunden.

Ferner wurden herbizide Mittel gefunden, welche die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I enthalten je nach Substitutionsmuster zwei oder mehr Chiralitätszentren und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di-(2-hydroxyeth-1-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R17 oder als Reste an Phenyl- oder Hetrocyclylringen genannten organischen Molekülteile stellen Sammelbegriffe für individuelle Aufzählungen der einzelnen Gruppenmitglieder dar. Sämtliche Kohlenwasserstoffketten, also z.B. alle Alkyl-, Alkenyl-, Alkinyl-, Cyanoalkyl-, Halogenalkyl-, Halogenalkenyl-, Halogenalkinyl-, Alkoxy-, Halogenalkoxy-, Alkoxyalkyl-, Alkylcarbonyl-, Alkenylcarbonyl-, Alkinylcarbonyl-, Alkoxycarbonyl-, Alkenyloxycarbonyl-, Alkinyloxycarbonyl-, Alkylamino-, Alkylaminocarbonyl-, Alkenylaminocarbonyl-, Alkinylaminocarbonyl-, Alkylsulfonylaminocarbonyl, Dialkylaminocarbonyl-, N-Alkenyl-N-alkylaminocarbonyl-, N-Alkinyl-N-alkylamino-carbonyl-, N-Alkoxy-N-alkylaminocarbonyl-, N-Alkenyl-N-alkoxyaminocarbonyl-, N-Alkinyl-N-alkoxyaminocarbonyl-, Dialkylaminothiocarbonyl, Alkylcarbonylalkyl, Alkoxyiminoalkyl, N-(Alkylamino)-iminoalkyl, N-(Dialkylamino)-iminoalkyl, Phenylalkyl-, Phenylcarbonylalkyl-, N-Alkyl-N-phenylaminocarbonyl-, Phenylalkylcarbonyl, Heterocyclylalkyl-, Heterocyclylcarbonylalkyl-, N-Alkyl-N-heterocyclylaminocarbonyl-, Heterocyclylalkylcarbonyl-, Alkylthio- und Alkylcarbonyloxy-Teile können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-₄-Alkyl sowie die Alkylteile von C₁-C₆-Alkyliminooxy-C₁-C₄-alkyl, Hydro-xy(C₁-C₄-alkyl), Tri(C₁-C₆-alkyl)silyloxy-C₁-C₄-alkyl, (C₁-C₄-Alkylcarbonyl)oxy-C₁-C₄-alkyl, Hydroxycarbonyl-C₁-C₄-alkoxy-C₁-C₄-alkyl, (C₁-C₄-Alkylsulfonyl)oxy-C₁-C₄-alkyl, C₁-C₄-Alkyl-O-C(O)-[C₁-C₄-alkyl-O]₃-C₁-C₄-alkyl, Carbamoyloxy-C₁-C₄-alkyl, (C₁-C₄-Alkylaminocarbonyl)oxy-C₁-C₄-alkyl, [Di(C₁-C₄-alkyl)aminocarbonyl]-oxy-C₁-C₄-alkyl, [(C₁-C₄-Halogenalkylsulfonyl)aminocarbonyl]oxy-C₁-C₄-alkyl, C₁-C₄-Alkylsulfonylamino, -C(O)-[C₁-C₄-alkyl-O]₃-C₁-C₄-alkyl, -C(O)-O-C₁-C₄-Alkyl-O-Phenyl: z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl sowie die Alkylteile von C₁-C₆-Alkylsulfonylaminocarbonyl, N-(C₃-C₆-Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkoxy)-N-(C₁-C₆-alkyl)-aminocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkyl-amino)-imino-C₁-C₆-alkyl, Phenyl-C₁-₆-alkyl, Phenylcarbonyl-C₁-C₆-alkyl, N-(C₁-C₆-Alkyl)-N-phenylaminocarbonyl, Heterocyclyl-C₁-C₆-alkyl, Hetrocyclyl-carbonyl-C₁-C₆-alkyl, N-(C₁-C₆-Alkyl)-N-heterocyclylaminocarbonyl, Tri(C₁-C₆-alkyl)silyloxy-C₁-C₄-alkyl, Tri(C₁-C₆-alkyl)silyl, (Hydroxycarbonyl)-C₁-C₆-alkyl, (C₁-C₆-Alkoxycarbonyl)-C₁-C₆-alkyl:
- C₁-C₄-Alkyl, wie voranstehend genannt, sowie z.B. n-Pentyl, 1-Methyl-butyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Alkylcarbonyl sowie die Alkylcarbonyl-Teile von (C₁-C₄-Alkylcarbonyl)oxy, (C₁-C₄-Alkylcarbonyl)oxy-C₁-C₄-alkyl, C₁-C₄-Alkylcarbonylamino: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonyl-C₁-C₆-alkyl, Phenyl-C₁-C₆-alkylcarbonyl und Heterocyclyl-C₁-C₆-alkylcarbonyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methyl-propylcarbonyl;
- C₃-C₆-Cycloalkyl sowie die Cycloalkylteile von C₃-C₆-Cycloalkylcarbonyl: monocyclischer, gesättigter Kohlenwasserstoff mit 3 bis 6 Ringgliedern, wie Cyclopropyl, Cyclobutyl, Cyclopentyl und Cyclohexyl;
- C₃-C₆-Alkenyl sowie die Alkenylteile von C₃-C₆-Alkenyloxycarbonyl, C₃-C₆-Alkenylaminocarbonyl, N-(C₃-C₆-Alkenyl)-N-(C₁-C₆-alkyl)aminocarbonyl und N-(C₃-C₆-Alkenyl)-N-(C₁-C₆-alkoxy)aminocarbonyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyl sowie die Alkenylteile von C₂-C₆-Alkenylcarbonyl, (Hydroxycarbonyl)-C₂-C₆-alkenyl, (C₁-C₆-Alkoxycarbonyl)-C₂-C₆-alkenyl: C₃-C₆-Alkenyl wie voranstehend genannt sowie Ethenyl;
- C₃-C₆-Alkinyl sowie die Alkinylteile von C₃-C₆-Alkinyloxycarbonyl, C₃-C₆-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₆-Alkinyl sowie die Alkinylteile von C₂-C₆-Alkinylcarbonyl: C₃-C₆-Alkinyl wie voranstehend genannt sowie Ethinyl;
- C₁-C₄-Cyanoalkyl: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl;
- C₁-C₄-Halogenalkyl sowie die Halogenalkylreste von [(C₁-C₄-Halogenalkylsulfonyl)aminocarbonyl]oxy-C₁-C₄-alkyl, C₁-C₄-Halogenalkylsulfonylamino, [(C₁-C₄-Halogenalkylsulfonyl)aminocarbonyl]amino: ein C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, lodmethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl sowie die Halogenalkylreste von C₁-C₆-Halogenalkyl-sulfonylaminocarbonyl, C₁-C₆-Halogenalkylthio: C₁-C₄-Halogenalkyl wie voranstehend genannt, sowie z.B. 5-Fluorpentyl, 5-Chlorpentyl, 5-Brompentyl, 5-lodpentyl, Undecafluorpentyl, 6-Fluorhexyl, 6-Chlorhexyl, 6-Bromhexyl, 6-lodhexyl und Dodecafluorhexyl;
- C₃-C₆-Halogenalkenyl: ein C₃-C₆-Alkenylrest, wie voranstehend genannt, der partiell oder vollständig durch Fluor, Chlor, Brom und/oder Iod substituiert ist, z.B. 2-Chlorprop-2-en-1-yl, 3-Chlorprop-2-en-1-yl, 2,3-Dichlorprop-2-en-1-yl, 3,3-Dichlorprop-2-en-1-yl, 2,3,3-Trichlor-2-en-1-yl, 2,3-Dichlorbut-2-en-1-yl, 2-Bromprop-2-en-1-yl, 3-Bromprop-2-en-1-yl, 2,3-Dibromprop-2-en-1-yl, 3,3-Dibromprop-2-en-1-yl, 2,3,3-Tribrom-2-en-1-yl oder 2,3-Dibrombut-2-en-1-yl;
- C₃-C₆-Halogenalkinyl: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2in-1-yl, 1,1-Difluorbut-2-in-1-yl, 4-lodbut-3-in-1-yl, 5-Fluorpent-3-in-1-yl, 5-lodpent-4-in-1-yl, 6-Fluorhex-4-in-1-yl oder 6-Iodhex-5-in-1-yl;
- C₁-C₄-Alkoxy sowie die Alkoxyteile von (C₁-C₄-Alkoxycarbonyl)-C₁-C₄-alkoxy, Hydroxycarbonyl-C₁-C₄-alkoxy-C₁-C₄-alkyl, (Hydroxycarbonyl)-C₁-C₄-alkoxy: z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy sowie die Alkoxyteile von N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl und C₁-C₆-Alkoxyimino-C₁-C₆-Alkyl: C₁-C₄-Alkoxy wie voranstehend genannt, sowie z.B. Pentoxy, 1-Methylbutoxy, 2-Methylbutoxy, 3-Methoxylbutoxy, 1,1-Dimethyl-propoxy, 1,2-Dimethyl-propoxy, 2,2-Dimethylpropoxy, 1-Ethylpropoxy, Hexoxy, 1-Methylpentoxy, 2-Me-thylpentoxy, 3-Methylpentoxy, 4-Methylpentoxy, 1,1-Di-methyl-butoxy,1,2-Dimethyl-butoxy, 1,3-Dimethylbutoxy, 2,2-Dimethylbutoxy, 2,3-Dimethylbutoxy, 3,3-Dimethyl-butoxy, 1-Ethylbutoxy, 2-Ethylbutoxy, 1,1,2-Trimethylpropoxy, 1,2,2-Trimethyl-propoxy, 1-Ethyl-1-methylpropoxy und 1-Ethyl-2-methylpropoxy;
- C₁-C₄-Halogenalkoxy: ein C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
   C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-lodpentoxy, Undecafluorpentoxy, 6-Fluorhexoxy, 6-Chlorhexoxy, 6-Bromhexoxy, 6-lodhexoxy und Dodecafluorhexoxy;
- C₁-C₆-Alkoxy-C₁-C₄-alkyl: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)-ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)-propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)-butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)-butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)-butyl;
- C₁-C₄-Alkoxycarbonyl sowie die Alkoxycarbonylteile von C₁-C₄-Alkoxy-C₁-C₄-alkoxycarbonyl, Di-(C₁-C₄-alkyl)-amino-C₁-C₇-alkoxycarbonyl, (C₁-C₄-Alkoxycarbonyl)-C₁-C₄-alkoxy: z.B. Methoxycarbonyl, Ethoxycarbonyl, Propoxycarbonyl, 1-Methylethoxycarbonyl, Butoxycarbonyl, 1-Methylpropoxycarbonyl, 2-Methylprop-oxycarbonyl oder 1,1-Dimethylethoxycarbonyl;
- C₁-C₆-Alkoxycarbonyl sowie die Alkoxycarbonylteile von (C₁-C₆-Alkoxycarbonyl)-C₁-C₆-alkyl, (C₁-C₆-Alkoxycarbonyl)-C₂-C₆-alkenyl: C₁-C₄-Alkoxycarbonyl, wie voranstehend genannt, sowie z.B. Pentoxycarbonyl, 1-Methylbutoxycarbonyl, 2-Methylbutoxycarbonyl, 3-Methyl-butoxycarbonyl, 2,2-Dimethylpropoxycarbonyl, 1-Ethylpropoxycarbonyl, Hexoxy-carbonyl, 1,1-Dimethylpropoxycarbonyl, 1,2-Dimethylpropoxycarbonyl, 1-Methyl-pentoxycarbonyl, 2-Methylpentoxycarbonyl, 3-Methylpentoxycarbonyl, 4-Methyl-pentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1- Ethyl-2-methylpropylthio;
- C₁-C₆-Alkylamino sowie die Alkylaminoreste von N-(C₁-C₆-Alkylamino)-imino-C₁-C₆-alkyl: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methyl-pentylamino, 4-Methylpentylamino, 1,1-Dimethylbutylamino, 1,2-Dimethylbutyl-amino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethyl-propylamino, 1,2,2-Trimethylpropylamino, 1-Ethyl-1-methylpropylamino oder 1-Ethyl-2-methylpropylamino;
- Di-(C₁-C₄-alkyl)-amino: z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)-amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethyl-ethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)-amino, N-Ethyl-N-(1,1-dimethyl-ethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethyl-ethyl)-N-propylamino, N-Butyl-N-(1-methylethyl-)amino, N-(1-Methylethyl)-N-(1-methylpropyl)amino, N-(1-Methylethyl)-N-(2-methyl-propyl)amino, N-(1,1-Dimethyl-ethyl)-N-(1-methylethyl)amino, N-Butyl-N-(1-methylpropyl)amino, N-Butyl-N-(2-methylpropyl)amino, N-Butyl-N-(1,1-dimethyl-ethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino sowie die Dialkylaminoreste von N-(Di-C₁-C₆-alkylamino)-imino-C₁-C₆-alkyl: Di-(C₁-C₄-alkyl)-amino wie voranstehend genannt sowie: z.B. N,N-Dipentyl-amino, N,N-Dihexylamino, N-Methyl-N-pentylamino, N-Ethyl-N-pentylamino, N-Methyl-N-hexylamino und N-Ethyl-N-hexylamino;
- (C₁-C₄-Alkylamino)carbonyl sowie die (Alkylamino)carbonyl-Teile von (C₁-C₄-Alkylaminocarbonyl)oxy-C₁-C₄-alkyl, (C₁-C₄-Alkylamino)carbonylamino: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di(C₁-C₄)alkylaminocarbonyl sowie die Di(C₁-C₄)alkylaminocarbonyl-Teile von [Di(C₁-C₄-alkyl)aminocarbonyl]oxy-C₁-C₄-alkyl, [Di(C₁-C₄-alkyl)amino]carbonyl-amino: z.B. N,N-Dimethylaminocarbonyl, N,N-Diethyl-aminocarbonyl, N,N-Di-(1-methylethyl)aminocarbonyl, N,N-Dipropylaminocarbonyl, N,N-Dibutylamino-carbonyl, N,N-Di-(1-methylpropyl)aminocarbonyl, N,N-Di-(2-methylpropyl)aminocarbonyl, N,N-Di-(1,1 -dimethylethyl)aminocarbonyl, N-Ethyl-N-methylaminocarbonyl, N-Methyl-N-propylaminocarbonyl, N-Methyl-N-(1-methyl-ethyl)aminocarbonyl, N-Butyl-N-methylaminocarbonyl, N-Methyl-N-(1-methyl--propyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)aminocarbonyl, N-Butyl-N-(1,1-dimethyl-ethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;
- (C₁-C₆-Alkylamino)carbonyl: (C₁-C₄-Alkylamino)carbonyl, wie voranstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylamino-carbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutyl-aminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)- aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)- aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethyl-propyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N,N-Di-(1-methylethyl)aminothiocarbonyl, N,N-Dipropyl-aminothiocarbonyl, N,N-Dibutylaminothiocarbonyl, N,N-Di-(1-methylpropyl)-amino-thiocarbonyl, N,N-Di-(2-methylpropyl)-aminothiocarbonyl, N,N-Di-(1,1-dimethyl-ethyl)-aminothiocarbonyl, N-Ethyl-N-methylaminothiocarbonyl, N-Methyl-N-propyl-aminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylamino-thiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1, 1-Dimethylethyl)-N-(2-methylpropyl)-aminothiocarbonyl, N-Methyl-N-pentylaminothiocarbonyl, N-Methyl-N-(1-methylbutyl)-aminothio-carbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothio-carbonyl, N-Methyl-N-(1-ethylpropyl)-aminothiocarbonyl, N-Methyl-N-hexyl-aminothiocarbonyl, N-Methyl-N- (1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothio-carbonyl, N-Methyl-N- (1,1-dimethylbutyl)-aminothiocarbonyl, N-Methy)-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentyl-aminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)- aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethyl-propyl)-aminothiocarbonyl, N-Ethyl-N-(1 ,2-dimethylpropyl)- aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-amino-thiocarbonyl, N-Ethyl-N-(1,2-dimethylbutyl)- aminothiocarbonyl, N-Ethyl-N-(1,3-dimethylbutyl)-aminothiocarbonyl, N-Ethyl-N-(2,2-dimethylbutyl)-aminothio-carbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexyl-aminothiocarbonyl, N-Butyl-N-hexylaminothiocarbonyl, N-Pentyl-N-hexyl-aminothiocarbonyl oder N,N-Dihexylaminothiocarbonyl;
- Heterocyclyl, sowie die Heterocyclylteile von Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl und Heterocyclyl-C₁-C₆-alkylcarbonyl:
   ein gesättigter, partiell ungesättigter oder aromatischer 5- oder 6-gliedriger heterocyclischer Ring, der ein bis vier gleiche oder verschiedene Heteroatome, ausgewählt aus der Gruppe Sauerstoff, Schwefel oder Stickstoff, enthält, und über C oder N gebunden sein kann, z.B.
   C-gebundene, 5-gliedrige, gesättigte Ringe wie
   Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
   N-gebundene, 5-gliedrige, gesättigte Ringe wie:
   Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl;
   C-gebundene, 5-gliedrige, partiell ungesättigte Ringe wie:
   2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1 H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydro-oxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxa-thiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadia-zolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1 ,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl;
   N-gebundene, 5-gliedrige, partiell ungesättigte Ringe wie:
   2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl;
   C-gebundene, 5-gliedrige, aromatische Ringe wie:
   2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   N-gebundene, 5-gliedrige, aromatische Ringe wie:
   Pyrrol-1-yl, Pyrazol-1-yl, Imidazol-1-yl, 1,2,3-Triazol-1-yl, 1,2,4-Triazol-1-yl, Tetrazol-1-yl;
   C-gebundene, 6-gliedrige, gesättigte Ringe wie:
   Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;
   N-gebundene, 6-gliedrige, gesättigte Ringe wie:
   Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;
   C-gebundene, 6-gliedrige, partiell ungesättigte Ringe wie:
   2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetra-hydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydro-thiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydro-pyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydro-pyridin-4-yl, 3,4-Dihydropyridin-5-yl, 3,4-Dihydropyridin-6-yl, 2,5-Dihydro-pyridin-2-yl, 2,5-Dihydropyridin-3-yl, 2,5-Dihydropyridin-4-yl, 2,5-Dihydro-pyridin-5-yl, 2,5-Dihydropyridin-6-yl, 2,3-Dihydropyridin-2-yl, 2,3-Dihydro-pyridin-3-yl, 2,3-Dihydropyridin-4-yl, 2,3-Dihydropyridin-5-yl, 2,3-Dihydro-pyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1 ,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Di-hydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin--4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydro-pyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-0xazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-0xazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;
   N-gebundene, 6-gliedrige, partiell ungesättigte Ringe wie:
   1,2,3,4-Tetrahydropyridin-1-yl, 1,2,5,6-Tetrahydropyridin-1-yl, 1,4-Dihydro-pyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
   C-gebundene, 6-gliedrige, aromatische Ringe wie:
   Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl;
   wobei mit einem ankondensierten Phenylring oder mit einem C₃-C₆-Carboxyclus oder mit einem weiteren 5- bis 6-gliedrigen Heterocyclus ein bicyclisches Ringsystem ausgebildet werden kann.

   Alle Phenylringe bzw. Heterocyclylreste sowie alle Phenylkomponenten in Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-phenylaminocarbonyl und Phenyl-C₁-C₆-alkylcarbonyl, und alle Heterocyclylkomponenten in Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-heterocyclylaminocarbonyl und Heterocyclyl-C₁-C₆-alkylcarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In einer besonderen Ausführungsform haben die Variablen der Verbindungen der Formel I folgende Bedeutungen, wobei diese für sich allein betrachtet als auch in Kombination miteinander besondere Ausgestaltungen der Verbindungen der Formel I darstellen:

Bevorzugt sind die benzoylsubstituierten Phenylalanin-Amide der Formel I, in der
- R¹: Halogen, C₁-C₄-Alkyl oder C₁-C₆-Halogenalkyl;
besonders bevorzugt Halogen oder C₁-C₆-Halogenalkyl;
insbesondere bevorzugt Halogen oder C₁-C₄-Halogenalkyl;
außerordentlich bevorzugt Fluor, Chlor oder CF₃;
bedeutet.

Ebenso bevorzugt sind die benzoylsubstituierten Phenylalanin-Amide der Formel I, in der
- R²: Wasserstoff, Halogen, NO₂, C₁-C₄-Alkyl oder C₁-C₆-Halogenalkyl;
sehr bevorzugt Wasserstoff, Halogen, NO₂ oder C₁-C₆-Halogenalkyl;
besonders bevorzugt Wasserstoff, Halogen, NO₂ oder C₁-C₄-Halogenalkyl;
insbesondere bevorzugt Wasserstoff, Fluor, Chlor, NO₂ oder CF₃;
außerordentlich bevorzugt Wasserstoff, Fluor, Chlor oder NO₂;
sehr außerordentlich bevorzugt Wasserstoff, Fluor oder NO₂;
bedeutet.

Ebenso bevorzugt sind die benzoyl substituierten Phenylalanin-Amide der Formel I, in der
- R² und R³: unabhängig voneinander
Wasserstoff, Halogen, C₁-C₄-Alkyl oder C₁-C₆-Halogenalkyl;
sehr bevorzugt Wasserstoff, Halogen oder C₁-C₆-Halogenalkyl;
besonders bevorzugt Wasserstoff, Halogen oder C₁-C₄-Halogenalkyl;
insbesondere bevorzugt Wasserstoff, Fluor, Chlor oder CF₃;
außerordentlich bevorzugt Wasserstoff, Fluor oder Chlor;
sehr außerordentlich bevorzugt Wasserstoff oder Fluor;
bedeuten.

Ebenso bevorzugt sind die benzoylsubstituierten Phenylalanin-Amide der Formel I, in der
- R⁴: Wasserstoff, Halogen, C₁-C₄-Alkyl oder C₁-C₄-Halogenalkyl;
besonders bevorzugt Wasserstoff, Halogen oder C₁-C₄-Alkyl;
insbesondere bevorzugt Wasserstoff oder Halogen;
außerordentlich bevorzugt Wasserstoff;
bedeutet.

Ebenso bevorzugt sind die benzoylsubstituierten Phenylalanin-Amide der Formel I, in der
- R⁵: Wasserstoff, Halogen, C₁-C₄-Alkyl oder C₁-C₄-Halogenalkyl ;
besonders bevorzugt Wasserstoff, Halogen oder C₁-C₄-Alkyl;
insbesondere bevorzugt Wasserstoff oder Halogen;
außerordentlich bevorzugt Wasserstoff;
bedeutet.

Ebenso bevorzugt sind die benzoylsubstituierten Phenylalanin-Amide der Formel I, in der
- R⁶: Wasserstoff; und
- R⁷: Wasserstoff oder Hydroxy;
besonders bevorzugt Wasserstoff;
bedeuten.

Ebenso bevorzugt sind die benzoylsubstituierten Phenylalanin-Amide der Formel I, in der
- R⁸: C₁-C₆-Alkyl oder C₁-C₆-Halogenalkyl;
besonders bevorzugt C₁-C₆-Alkyl;
insbesondere bevorzugt C₁-C₄-Alkyl;
außerordentlich bevorzugt CH₃;
bedeutet.

Ebenso bevorzugt sind die benzoylsubstituierten Phenylalanin-Amide der Formel I, in der
- R¹⁰: Wasserstoff oder C₁-C₄-Alkyl;
bevorzugt Wasserstoff oder CH₃;
insbesondere bevorzugt Wasserstoff;
bedeutet.

Ebenso bevorzugt sind die benzoylsubstituierten Phenylalonin-Amide der Formel I, in der
- R¹¹: Wasserstoff, Halogen, C₁-C₆-Alkyl, Hydroxy, C₁-C₆-Alkoxy, Hydroxy-C₁-C₄-alkyl, C₁-C₆-Alkoxy-C₁-C₄-alkyl, Tri(C₁-C₆-alkyl)silyloxy-C₁-C₄-alkyl, (Hydroxycarbonyl)-C₁-C₄-alkoxy, (C₁-C₄-Alkoxycarbonyl)-C₁-C₄-alkoxy, [Di(C₁-C₄-alkyl)aminocarbonyl]oxy-C₁-C₄-alkyl, C₁-C₄-Alkylsulfonylamino, C₁-C₄-Halogenalkylsulfonylamino, (C₁-C₄-Alkylcarbonyl)amino oder Phenyl, wobei der Phenylrest ein bis drei Reste aus folgender Gruppe tragen kann: Halogen, Nitro, C₁-C₄-Alkyl, C₁-C₄-Halogenalkyl, Hydroxycarbonyl und C₁-C₆-Alkoxycarbonyl;
besonders bevorzugt Wasserstoff, Halogen, C₁-C₆-Alkyl, Hydroxy-C₁-C₄-alkyl, (Hydroxycarbonyl)-C₁-C₄-alkoxy, (C₁-C₄-Alkoxycarbonyl)-Ci -C₄-alkoxy oder (C₁-C₄-Alkylcarbonyl)amino;
insbesondere bevorzugt Wasserstoff, Halogen, C₁-C₄-Alkyl, Hydroxy-C₁-C₄-alkyl, (Hydroxycarbonyl)-C₁-C₄-alkoxy, (C₁-C₄-Alkoxycarbonyl)-C₁-C₄-alkoxy oder (C₁-C₄-Alkylcarbonyl)amino;
sehr bevorzugt Wasserstoff, Fluor, Chlor, Brom, CH₃, Hydroxy-C₁-C₄-alkyl, (Hydroxycarbonyl)-C₁-C₄-alkoxy, (C₁-C₄-Alkoxycarbonyl)-C₁-C₄-alkoxy oder (C₁-C₆-Alkylcarbonyl)amino;
außerordentlich bevorzugt Wasserstoff, Fluor, CH₃, Hydroxy-C₁-C₄-alkyl, (Hydroxycarbonyl)-C₁-C₄-alkoxy, (C₁-C₄-Alkoxycarbonyl)-C₁-C₄-alkoxy oder (C₁-C₆-Alkylcarbonyl)amino;
bedeutet.

Ebenso bevorzugt sind die benzoylsubstituierten Phenylalanin-Amide der Formel I, in der
- R¹¹: Wasserstoff, Halogen, C₁-C₆-Alkyl, Hydroxy oder C₁-C₆-Alkoxy;
besonders bevorzugt Wasserstoff, Halogen oder C₁-C₆-Alkyl;
insbesondere bevorzugt Wasserstoff, Halogen oder C₁-C₄-Alkyl;
sehr bevorzugt Wasserstoff, Fluor, Chlor, Brom oder CH₃;
außerordentlich bevorzugt Wasserstoff, Fluor oder CH₃;
bedeutet.

Ebenso bevorzugt sind die benzoylsubstituierten Phenylalanin-Amide der Formel I, in der
- R¹²: Wasserstoff, Halogen, C₁-C₆-Alkyl, C₁-C₆-Halogenalkyl oder (C₁-C₄-Alkylcarbonyl)amino;
besonders bevorzugt Wasserstoff, Halogen, C₁-C₆-Alkyl oder (C₁-C₄-Alkylcarbonyl)amino;
insbesondere bevorzugt Wasserstoff, Halogen, C₁-C₄-Alkyl oder (C₁-C₄-Alkylcarbonyl)amino;
bedeutet.

Ebenso bevorzugt sind die benzoylsubstituierten Phenylalanin-Amide der Formel I, in der
- R¹²: Wasserstoff, Halogen, C₁-C₆-Alkyl oder C₁-C₆-Halogenalkyl;
besonders bevorzugt Wasserstoff, Halogen oder C₁-C₆-Alkyl;
insbesonders bevorzugt Wasserstoff, Halogen oder C₁-C₄-Alkyl;
außerordenrlich bevorzugt Wasserstoff oder Halogen;
sehr außerordentlich bevorzugt Wasserstoff, Fluor oder Chlor;
bedeutet.

Ebenso bevorzugt sind die benzoylsubstituierten Phenylalanin-Amide der Formel I, in der
- R¹³, R¹⁴ und R¹⁵: jeweils unabhängig voneinander
Wasserstoff, Halogen, Cyano, C₁-C₄-Alkyl oder C₁-C₄-Halogenalkyl;
besonders bevorzugt Wasserstoff, Halogen oder Cyano;
insbesondere bevorzugt Wasserstoff, Fluor oder Chlor;
außerordentlich bevorzugt Wasserstoff;
bedeuten.

Ebenso bevorzugt sind die benzoylsubstituierten Phenylalanin-Amide der Formel I, in der
- R¹⁶: Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, C₁-C₆-Alkylsulfonylaminocarbonyl, Di-(C₁-C₆-alkyl)-aminocarbonyl, N-(C₁-C₆-A)koxy)-N-(C₁-C₆-alkyl)-aminocarbonyl, Di-(C₁-C₆-alkyl)-aminothicarbonyl, C₁-C₆-Alkoxyimino-C₁-C₆-alkyl,
wobei die genannten Alkyl, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl, oder C₁-C₄-Alkylcarbonyloxy;
Phenyl, Phenyl-C₁-C₆-alkyl, Phenylcarbonyl, Phenylcarbonyl-C₁-C₆-alkyl, Phenylsulfonylaminocarbonyl oder Phenyl-C₁-C₆-alkylcarbonyl,
wobei der Phenylrest der 6 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹⁷;
besonders bevorzugt Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, C₁-C₆-Alkylsulfonylaminocarbonyl, Di-(C₁-C₆-alkyl)-aminocarbonyl, N-(C₁-C₆-Alkoxy)-N-(C₁-C₆-alkyl)-aminocarbonyl oder Di-(C₁-C₆-alkyl)-aminothiocarbonyl,
wobei die genannten Alkyl- oder Alkoxyreste partiell oder vollständig halogeniert sein können und/oder eine bis drei der folgenden Gruppen tragen können: Cyano, C₁-C₄-Alkoxy, C₁-C₄-Alkoxycarbonyl , C₁-C₄-Alkylaminocarbonyl, Di-(C₁-C₄-alkyl)-aminocarbonyl oder C₁-C₄-Alkylcarbonyloxy;
Phenyl-C₁-C₆-alkyl, Phenylcarbonyl, Phenylcarbonyl-C₁-C₆-alkyl, Phenylsulfonylaminocarbonyl oder Phenyl-C₁-C₆-alkylcarbonyl,
wobei der Phenylring der 5 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenoxy; oder
SO₂R¹⁷;
insbesondere bevorzugt Wasserstoff, C₁-C₆-Alkyl, C₃-C₆-Alkenyl, C₃-C₆-Alkinyl, C₁-C₆-Alkyl-carbonyl, C₂-C₆-Alkenylcarbonyl, C₁-C₆-Alkoxycarbonyl, Di-(C₁-C₆-alkyl)-amino-carbonyl, N-(C₁-C₆-Alkoxy)-N-(C₁-C₆-alkyl)-aminocarbonyl, Di-(C₁-C₆-alkyl)-aminothiocarbonyl, Phenyl-C₁-C₆-alkyl, Phenylcarbonyl, Phenylcarbonyl-C₁-C₆-alkyl oder Phenyl-C₁-C₆-alkylcarbonyl
wobei der Phenylring der 4 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogen-alkoxy; oder
SO₂R¹⁷;
bedeuten.

Ebenso bevorzugt sind die benzoylsubstituierten Phenylalanin-Amide der Formel I, in der
- R¹⁶: Wasserstoff, C₁-C₆-Alkyl, C₃-C₆-Alkenyl, C₃-C₆-Alkinyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothiocarbonyl, C₁-C₆-Alkoxyimino-C₁-C₆-alkyl, wobei die genannten Alkyl-, Cycloalkyl- oder Alkoxyreste partiell oder vollständig halogeniert sein können und/oder eine bis drei der folgenden Gruppen tragen können:
Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl oder C₁-C₄-Alkylcarbonyloxy; oder
SO₂R¹⁷;
bedeuten.

Ebenso bevorzugt sind die benzoylsubstituierten Phenylalanin-Amide der Formel I, in der
- R¹⁶: Wasserstoff, C₁-C₆-Alkyl, C₃-C₆-Alkenyl, C₃-C₆-Alkinyl, C₁-C₆-Alkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-amino-carbonyl,
wobei die genannten Alkyl-,und Alkoxyreste partiell oder vollständig halogeniert sein können und/oder eine bis drei der folgenden Gruppen tragen können: Cyano, C₁-C₄-Alkoxy, C₁-C₄-Alkylaminocarbonyl oder Di-(C₁-C₄-alkyl)-aminocarbonyl;
Phenyl-C₁-C₆-alkyl, Phenylcarbonyl, Phenylcarbonyl-Cᵢ-C₆-alkyl, Phenylaminocarbonyl, N-(C₁-C₆-Alkyl)-N-(phenyl)-aminocarbonyl oder Heterocyclylcarbonyl, wobei der Phenyl- und der Heterocyclylrest der 6 letztgenannten Substituenten partiell oder vollständig halogeniert sein kann und/oder eine bis drei der folgenden Gruppen tragen kann: Cyano, C₁-C₄-Alkyl oder C₁-C₄-Halogenalkyl; oder
SO₂R¹⁷;
besonders bevorzugt Wasserstoff, C₁-C₄-Alkyl, C₃-C₄-Alkenyl, C₃-C₄-Alkinyl, C₁-C₄-Alkylcarbonyl, C₁-C₄-Alkoxycarbonyl, C₁-C₄-Alkylaminocarbonyl, Di-(C₁-C₄-alkyl)-aminocarbonyl, N-(C₁-C₄-Alkoxy)-N-(C₁-C₄-alkyl)-aminocarbonyl,
wobei die genannten Alkyl-,und Alkoxyreste partiell oder vollständig halogeniert sein können und/oder eine bis drei der folgenden Gruppen tragen können: Cyano, C₁-C₄-Alkoxy, C₁-C₄-Alkylaminocarbonyl oder Di-(C₁-C₄-alkyl)-aminocarbonyl;
Phenyl-C₁-C₄-alkyl, Phenylcarbonyl, Phenylcarbonyl-C₁-C₄-alkyl, Phenylaminocarbonyl, N-(C₁-C₄-Alkyl)-N-(phenyl)-aminocarbonyl oder Heterocyclylcarbonyl,
wobei der Phenyl- und der Heterocyclylrest der 6 letztgenannten Substituenten partiell oder vollständig halogeniert sein kann und/oder eine bis drei der folgenden Gruppen tragen kann: Cyano, C₁-C₄-Alkyl oder C₁-C₄-Halogenalkyl; oder
SO₂R¹⁷;
insbesondere bevorzugt Wasserstoff oder C₁-C₄-Alkyl,
wobei der genannte Alkylrest partiell oder vollständig halogeniert sein kann und/oder eine bis drei der folgenden Gruppen tragen kann: Cyano, C₁-C₄-Alkoxy, C₁-C₄-Alkylamino-carbonyl oder Di-(C₁-C₄-alkyl)-aminocarbonyl;
Phenyl-C₁-C₄-alkyl, Phenylcarbonyl, Phenylcarbonyl-C₁-C₄-alkyl, Phenylaminocarbonyl, N-(C₁-C₄-Alkyl)-N-(phenyl)-aminocarbonyl oder Heterocyclylcarbonyl, oder
SO₂R¹⁷;
außerordentlich bevorzugt Wasserstoff, C₁-C₄-Alkylcarbonyl, C₁-C₆-Alkylaminocarbonyl, Di(C₁-C₄-alkyl)aminocarbonyl, Phenylaminocarbonyl, N(C₁-C₄-alkyl)-N(phenyl)aminocarbonyl, SO₂CH₃, SO₂CF₃ oder SO₂(C₆H₅);
bedeuten.

Ebenso bevorzugt sind die benzoylsubstituierten Phenylalanin-Amide der Formel I, in der
- R¹⁷: C₁-C₆-Alkyl, C₁-C₆-Halogenalkyl oder Phenyl, wobei der Phenylrest partiell oder teilweise halogeniert sein kann und/oder durch C₁-C₄-Alkyl substituiert sein kann;
besonders bevorzugt C₁-C₄-Alkyl, C₁-C₄-Halogenalkyl oder Phenyl;
insbesondere bevorzugt Methyl, Trifluormethyl oder Phenyl.
bedeutet.

Ebenso bevorzugt sind die benzoylsubstituierten Phenylalanin-Amide der Formel I, in der
- R¹: Fluor, Chlor oder CF₃,
- R² und R³: unabhängig voneinander Wasserstoff, Fluor oder Chlor,
- R⁴, R⁵, R⁶ und R⁷: Wasserstoff,
- R⁸: C₁-C₄-Alkyl, besonders bevorzugt CH₃;
- R⁹: OR¹⁶;
- R¹⁰: Wasserstoff;
- R¹¹: Wasserstoff, Halogen, Cyano oder C₁-C₄-Alkyl, besonders bevorzugt Wasserstoff, Fluor oder CH₃;
- R¹²: Wasserstoff, Halogen oder Cyano, besonders bevorzugt Wasserstoff, Fluor oder Chlor;
- R¹³, R¹⁴ und R¹⁵: unabhängig voneinander Wasserstoff, Fluor oder Chlor, besonders bevorzugt Wasserstoff; und
- R¹⁶: Wasserstoff, C₁-C₄-Alkylcarbonyl, C₁-C₄-Alkylaminocarbonyl, Di-(C₁-C₄-alkyl)-aminocarbonyl, Phenylaminocarbonyl, N-(C₁-C₄-alkyl)-N-(phenyl)-aminocarbonyl, SO₂CH₃, SO₂CF₃ oder SO₂(C₆H₅);
bedeuten.

Außerordentlich bevorzugt sind die Verbindungen der Formel I.a.1 (entspricht Formel I mit R¹= F; R², R³, R⁴, R⁵, R⁶, R⁷, R¹⁰, R¹⁴, R¹⁵ = H; R⁸ = CH₃), insbesondere die Verbindungen der Formel I.a.1.1 bis I.a.1.144 der Tabelle 1, wobei die Definitionen der Variablen R¹ bis R²⁰ nicht nur in Kombination miteinander sondern auch jeweils für sich allein betrachtet für die erfindungsgemäßen Verbindungen eine besondere Rolle spielen.

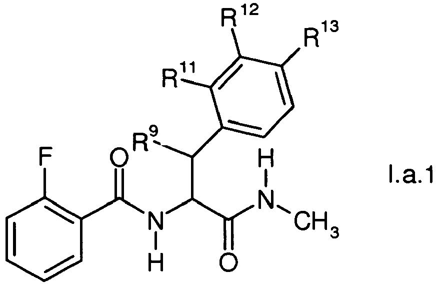

**Tabelle 1**

| Nr. | R⁹ | R¹¹ | R¹² | R¹³ |
|---|---|---|---|---|
| I.a.1.1 | OH | H | H | H |
| I.a.1.2 | OH | H | H | F |
| I.a.1.3 | OH | H | F | H |
| I.a.1.4 | OH | H | F | F |
| I.a.1.5 | OH | H | Cl | H |
| I.a.1.6 | OH | H | Cl | F |
| I.a.1.7 | OH | F | H | H |
| I.a.1.8 | OH | F | H | F |
| I.a.1.9 | OH | F | F | H |
| I.a.1.10 | OH | F | F | F |
| I.a.1.11 | OH | F | Cl | H |
| I.a.1.12 | OH | F | Cl | F |
| I.a.1.13 | OH | CH₃ | H | H |
| I.a.1.14 | OH | CH₃ | H | F |
| I.a.1.15 | OH | CH₃ | F | H |
| I.a.1.16 | OH | CH₃ | F | F |
| I.a.1.17 | OH | CH₃ | Cl | H |
| I.a.1.18 | OH | CH₃ | Cl | F |
| I.a.1.19 | OC(O)CH₃ | H | H | H |
| I.a.1.20 | OC(O)CH₃ | H | H | F |
| I.a.1.21 | OC(O)CH₃ | H | F | H |
| I.a.1.22 | OC(O)CH₃ | H | F | F |
| I.a.1.23 | OC(O)CH₃ | H | Cl | H |
| I.a.1.24 | OC(O)CH₃ | H | Cl | F |
| I.a.1.25 | OC(O)CH₃ | F | H | H |
| I.a.1.26 | OC(O)CH₃ | F | H | F |
| I.a.1.27 | OC(O)CH₃ | F | F | H |
| I.a.1.28 | OC(O)CH₃ | F | F | F |
| I.a.1.29 | OC(O)CH₃ | F | Cl | H |
| I.a.1.30 | OC(O)CH₃ | F | Cl | F |
| I.a.1.31 | OC(O)CH₃ | CH₃ | H | H |
| I.a.1.32 | OC(O)CH₃ | CH₃ | H | F |
| I.a.1.33 | OC(O)CH₃ | CH₃ | F | H |
| I.a.1.34 | OC(O)CH₃ | CH₃ | F | F |
| I.a.1.35 | OC(O)CH₃ | CH₃ | Cl | H |
| I.a.1.36 | OC(O)CH₃ | CH₃ | Cl | F |
| I.a.1.37 | OC(O)*tert*C₄H₉ | H | H | H |
| I.a.1.38 | OC(O)*tert*C₄H₉ | H | H | F |
| I.a.1.39 | OC(O)*tert*C₄H₉ | H | F | H |
| I.a.1.40 | OC(O)*tert*C₄H₉ | H | F | F |
| I.a.1.41 | OC(O)*tert*C₄H₉ | H | Cl | H |
| I.a.1.42 | OC(O)*tert*C₄H₉ | H | Cl | F |
| I.a.1.43 | OC(O)*tert*C₄H₉ | F | H | H |
| I.a.1.44 | OC(O)*tert*C₄H₉ | F | H | F |
| I.a.1.45 | OC(O)*tert*C₄H₉ | F | F | H |
| I.a.1.46 | OC(O)*tert*C₄H₉ | F | F | F |
| I.a.1.47 | OC(O)*tert*C₄H₉ | F | Cl | H |
| I.a.1.48 | OC(O)*tert*C₄H₉ | F | Cl | F |
| I.a.1.49 | OC(O)*tert*C₄H₉ | CH₃ | H | H |
| I.a.1.50 | OC(O)*tert*C₄H₉ | CH₃ | H | F |
| I.a.1.51 | OC(O)*tert*C₄H₉ | CH₃ | F | H |
| I.a.1.52 | OC(O)*tert*C₄H₉ | CH₃ | F | F |
| I.a.1.53 | OC(O)*tert*C₄H₉ | CH₃ | Cl | H |
| I.a.1.54 | OC(O)*tert*C₄H₉ | CH₃ | Cl | F |
| I.a.1.55 | OC(O)NH(CH₃) | H | H | H |
| I.a.1.56 | OC(O)NH(CH₃) | H | H | F |
| I.a.1.57 | OC(O)NH(CH₃) | H | F | H |
| I.a.1.58 | OC(O)NH(CH₃) | H | F | F |
| I.a.1.59 | OC(O)NH(CH₃) | H | Cl | H |
| I.a.1.60 | OC(O)NH(CH₃) | H | Cl | F |
| I.a.1.61 | OC(O)NH(CH₃) | F | H | H |
| I.a.1.62 | OC(O)NH(CH₃) | F | H | F |
| I.a.1.63 | OC(O)NH(CH₃) | F | F | H |
| I.a.1.64 | OC(O)NH(CH₃) | F | F | F |
| I.a.1.65 | OC(O)NH(CH₃) | F | Cl | H |
| I.a.1.66 | OC(O)NH(CH₃) | F | Cl | F |
| I.a.1.67 | OC(O)NH(CH₃) | CH₃ | H | H |
| I.a.1.68 | OC(O)NH(CH₃) | CH₃ | H | F |
| I.a.1.69 | OC(O)NH(CH₃) | CH₃ | F | H |
| I.a.1.70 | OC(O)NH(CH₃) | CH₃ | F | F |
| I.a.1.71 | OC(O)NH(CH₃) | CH₃ | Cl | H |
| I.a.1.72 | OC(O)NH(CH₃) | CH₃ | Cl | F |
| I.a.1.73 | OC(O)NH(C₆H₅) | H | H | H |
| I.a.1.74 | OC(O)NH(C₆H₅) | H | H | F |
| I.a.1.75 | OC(O)NH(C₆H₅) | H | F | H |
| I.a.1.76 | OC(O)NH(C₆H₅) | H | F | F |
| I.a.1.77 | OC(O)NH(C₆H₅) | H | Cl | H |
| I.a.1.78 | OC(O)NH(C₆H₅) | H | Cl | F |
| I.a.1.79 | OC(O)NH(C₆H₅) | F | H | H |
| I.a.1.80 | OC(O)NH(C₆H₅) | F | H | F |
| I.a.1.81 | OC(O)NH(C₆H₅) | F | F | H |
| I.a.1.82 | OC(O)NH(C₆H₅) | F | F | F |
| I.a.1.83 | OC(O)NH(C₆H₅) | F | Cl | H |
| I.a.1.84 | OC(O)NH(C₆H₅) | F | Cl | F |
| I.a.1.85 | OC(O)NH(C₆H₅) | CH₃ | H | H |
| I.a.1.86 | OC(O)NH(C₆H₅) | CH₃ | H | F |
| I.a.1.87 | OC(O)NH(C₆H₅) | CH₃ | F | H |
| I.a.1.88 | OC(O)NH(C₆H₅) | CH₃ | F | F |
| I.a.1.89 | OC(O)NH(C₆H₅) | CH₃ | Cl | H |
| I.a.1.90 | OC(O)NH(C₆H₅) | CH₃ | Cl | F |
| I.a.1.91 | OC(O)N(CH₃)₂ | H | H | H |
| I.a.1.92 | OC(O)N(CH₃)₂ | H | H | F |
| I.a.1.93 | OC(O)N(CH₃)₂ | H | F | H |
| I.a.1.94 | OC(O)N(CH₃)₂ | H | F | F |
| I.a.1.95 | OC(O)N(CH₃)₂ | H | Cl | H |
| I.a.1.96 | OC(O)N(CH₃)₂ | H | Cl | F |
| I.a.1.97 | OC(O)N(CH₃)₂ | F | H | H |
| I.a.1.98 | OC(O)N(CH₃)₂ | F | H | F |
| I.a.1.99 | OC(O)N(CH₃)₂ | F | F | H |
| I.a.1.100 | OC(O)N(CH₃)₂ | F | F | F |
| I.a.1.101 | OC(O)N(CH₃)₂ | F | Cl | H |
| I.a.1.102 | OC(O)N(CH₃)₂ | F | Cl | F |
| I.a.1.103 | OC(O)N(CH₃)₂ | CH₃ | H | H |
| I.a.1.104 | OC(O)N(CH₃)₂ | CH₃ | H | F |
| I.a.1.105 | OC(O)N(CH₃)₂ | CH₃ | F | H |
| I.a.1.106 | OC(O)N(CH₃)₂ | CH₃ | F | F |
| I.a.1.107 | OC(O)N(CH₃)₂ | CH₃ | Cl | H |
| I.a.1.108 | OC(O)N(CH₃)₂ | CH₃ | Cl | F |
| I.a.1.109 | OC(O)N(CH₃)(C₆H₅) | H | H | H |
| I.a.1.110 | OC(O)N(CH₃)(C₆H₅) | H | H | F |
| I.a.1.111 | OC(O)N(CH₃)(C₆H₅) | H | F | H |
| I.a.1.112 | OC(O)N(CH₃)(C₆H₅) | H | F | F |
| I.a.1.113 | OC(O)N(CH₃)(C₆H₅) | H | Cl | H |
| I.a.1.114 | OC(O)N(CH₃)(C₆H₅) | H | Cl | F |
| I.a.1.115 | OC(O)N(CH₃)(C₆H₅) | F | H | H |
| I.a.1.116 | OC(O)N(CH₃)(C₆H₅) | F | H | F |
| I.a.1.117 | OC(O)N(CH₃)(C₆H₅) | F | F | H |
| I.a.1.118 | OC(O)N(CH₃)(C₆H₅) | F | F | F |
| I.a.1.119 | OC(O)N(CH₃)(C₆H₅) | F | Cl | H |
| I.a.1.120 | OC(O)N(CH₃)(C₆H₅) | F | Cl | F |
| I.a.1.121 | OC(O)N(CH₃)(C₆H₅) | CH₃ | H | H |
| I.a.1.122 | OC(O)N(CH₃)(C₆H₅) | CH₃ | H | F |
| I.a.1.123 | OC(O)N(CH₃)(C₆H₅) | CH₃ | F | H |
| I.a.1.124 | OC(O)N(CH₃)(C₆H₅) | CH₃ | F | F |
| I.a.1.125 | OC(O)N(CH₃)(C₆H₅) | CH₃ | Cl | H |
| I.a.1.126 | OC(O)N(CH₃)(C₆H₅) | CH₃ | Cl | F |
| I.a.1.127 | OSO₂CH₃ | H | H | H |
| I.a.1.128 | OSO₂CH₃ | H | H | F |
| I.a.1.129 | OSO₂CH₃ | H | F | H |
| I.a.1.130 | OSO₂CH₃ | H | F | F |
| I.a.1.131 | OSO₂CH₃ | H | Cl | H |
| I.a.1.132 | OSO₂CH₃ | H | Cl | F |
| I.a.1.133 | OSO₂CH₃ | F | H | H |
| I.a.1.134 | OSO₂CH₃ | F | H | F |
| I.a.1.135 | OSO₂CH₃ | F | F | H |
| I.a.1.136 | OSO₂CH₃ | F | F | F |
| I.a.1.137 | OSO₂CH₃ | F | Cl | H |
| I.a.1.138 | OSO₂CH₃ | F | Cl | F |
| I.a.1.139 | OSO₂CH₃ | CH₃ | H | H |
| I.a.1.140 | OSO₂CH₃ | CH₃ | H | F |
| I.a.1.141 | OSO₂CH₃ | CH₃ | F | H |
| I.a.1.142 | OSO₂CH₃ | CH₃ | F | F |
| I.a.1.143 | OSO₂CH₃ | CH₃ | Cl | H |
| I.a.1.144 | OSO₂CH₃ | CH₃ | Cl | F |

Ebenso außerordentlich bevorzugt sind die Verbindungen der Formel I.a.2, insbesondere die Verbindungen der Formel I.a.2.1 bis I.a.2.144, die sich von den entsprechenden Verbindungen der Formel I.a.1.1 bis I.a.1.144 dadurch unterscheiden, daß R² für CF₃ steht.

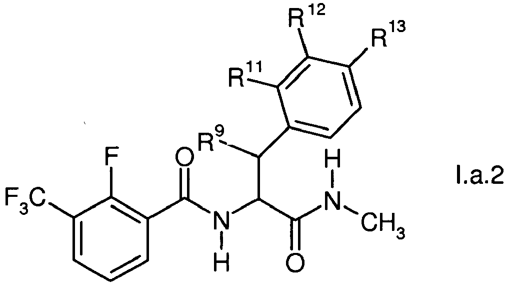

Ebenso außerordentlich bevorzugt sind die Verbindungen der Formel I.a.3, insbesondere die Verbindungen der Formel I.a.3.1 bis I.a.3.144, die sich von den entsprechenden Verbindungen der Formel I.a.1.1 bis I.a.1.144 dadurch unterscheiden, daß R² für CF₃ und R³ für Fluor steht.

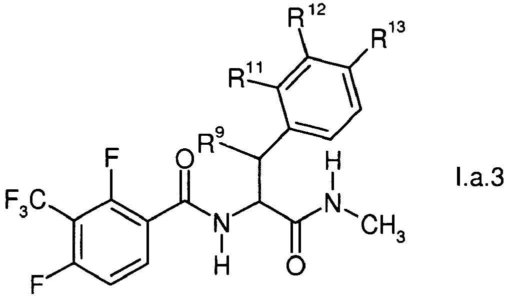

Ebenso außerordentlich bevorzugt sind die Verbindungen der Formel I.a.4, insbesondere die Verbindungen der Formel I.a.4.1 bis I.a.4.144, die sich von den entsprechenden Verbindungen der Formel I.a.1.1 bis I.a.1.144 dadurch unterscheiden, daß R² für CF₃ und R³ für Chlor steht.

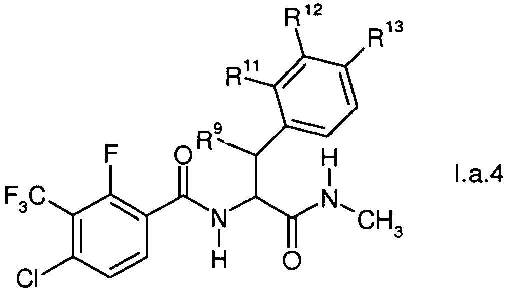

Ebenso außerordentlich bevorzugt sind die Verbindungen der Formel I.a.5, insbesondere die Verbindungen der Formel I.a.5.1 bis I.a.5.144, die sich von den entsprechenden Verbindungen der Formel I.a.1.1 bis I.a.1.144 dadurch unterscheiden, daß R¹ für Chlor und R³ für Fluor steht.

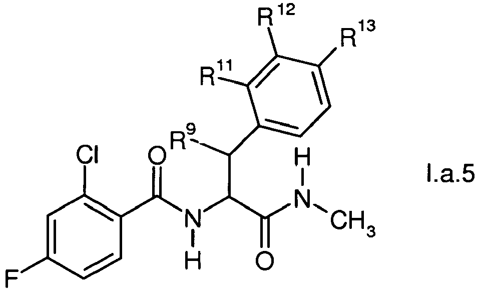

Ebenso außerordentlich bevorzugt sind die Verbindungen der Formel I.a.6, insbesondere die Verbindungen der Formel I.a.6.1 bis I.a.6.144, die sich von den entsprechenden Verbindungen der Formel I.a.1.1 bis I.a.1.144 dadurch unterscheiden, daß R¹ und R³ für Chlor stehen.

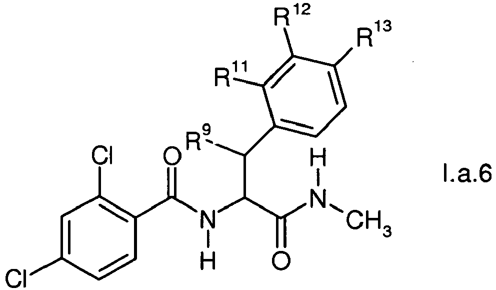

Ebenso außerordentlich bevorzugt sind die Verbindungen der Formel I.a.7, insbesondere die Verbindungen der Formel I.a.7.1 bis I.a.7.144, die sich von den entsprechenden Verbindungen der Formel I.a.1.1 bis I.a.1.144 dadurch unterscheiden, daß R¹ für Chlor und R² für Fluor steht.

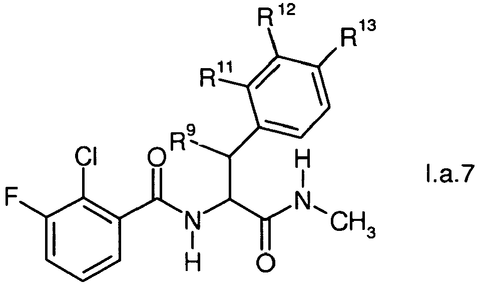

Ebenso außerordentlich bevorzugt sind die Verbindungen der Formel I.a.8, insbesondere die Verbindungen der Formel I.a.8.1 bis I.a.8.144, die sich von den entsprechenden Verbindungen der Formel I.a.1.1 bis I.a.1.144 dadurch unterscheiden, daß R1 für Chlor und R² sowie R³ für Fluor stehen.

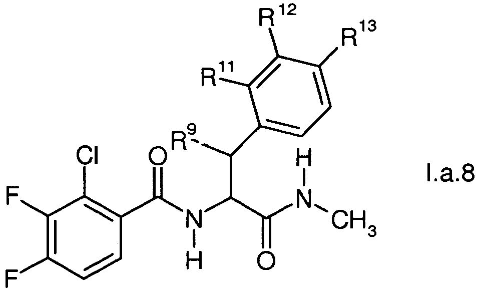

Ebenso außerordentlich bevorzugt sind die Verbindungen der Formel I.a.9, insbesondere die Verbindungen der Formel I.a.9.1 bis I.a.9.144, die sich von den entsprechenden Verbindungen der Formel I.a.1.1 bis I.a.1.144 dadurch unterscheiden, daß R¹ und R³ für Chlor und R² für Fluor steht.

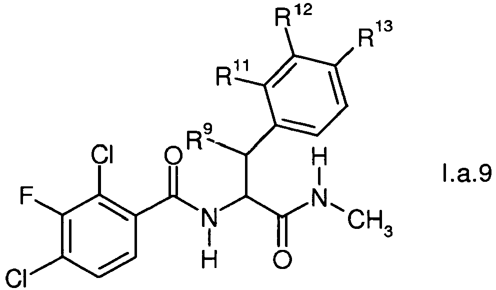

Ebenso außerordentlich bevorzugt sind die Verbindungen der Formel I.a.10, insbesondere die Verbindungen der Formel I.a.10.1 bis I.a.10.144, die sich von den entsprechenden Verbindungen der Formel I.a.1.1 bis I.a.1.144 dadurch unterscheiden, daß R¹ und R² für Chlor stehen.

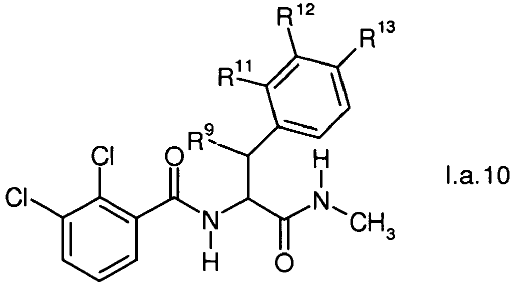

Ebenso außerordentlich bevorzugt sind die Verbindungen der Formel I.a.11, insbesondere die Verbindungen der Formel I.a.11.1 bis I.a.11.144, die sich von den entsprechenden Verbindungen der Formel I.a.1.1 bis I.a.1.144 dadurch unterscheiden, daß R¹ und R² für Chlor und R³ für Fluor stehen.

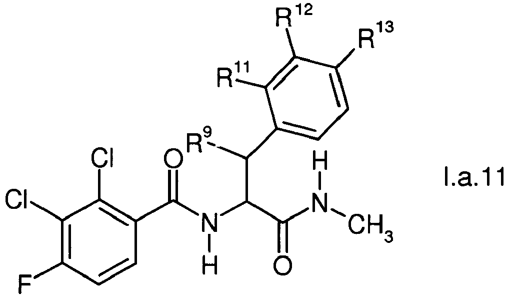

Ebenso außerordentlich bevorzugt sind die Verbindungen der Formel I.a.12, insbesondere die Verbindungen der Formel I.a.12.1 bis I.a.12.144, die sich von den entsprechenden Verbindungen der Formel I.a.1.1 bis I.a.1.144 dadurch unterscheiden, daß R¹, R² und R³ für Chlor stehen.

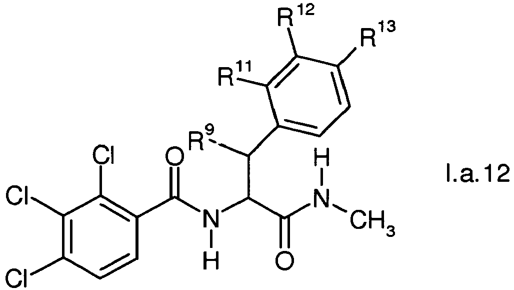

Ebenso außerordentlich bevorzugt sind die Verbindungen der Formel I.a.13, insbesondere die Verbindungen der Formel I.a.13.1 bis I.a.13.144, die sich von den entsprechenden Verbindungen der Formel I.a.1.1 bis I.a.1.144 dadurch unterscheiden, daß R¹ für Chlor und R² für CF₃ steht.

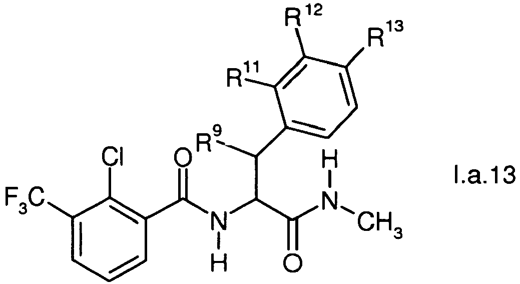

Ebenso außerordentlich bevorzugt sind die Verbindungen der Formel I.a.14, insbesondere die Verbindungen der Formel I.a.14.1 bis I.a.14.144, die sich von den entsprechenden Verbindungen der Formel I.a.1.1 bis I.a.1.144 dadurch unterscheiden, daß R¹ für Chlor, R² für CF₃ und R³ für Fluor steht.

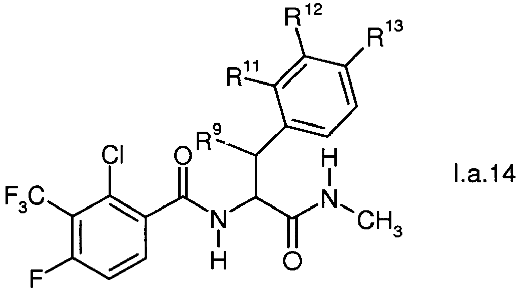

Ebenso außerordentlich bevorzugt sind die Verbindungen der Formel I.a.15, insbesondere die Verbindungen der Formel I.a.15.1 bis I.a.15.144, die sich von den entsprechenden Verbindungen der Formel I.a.1.1 bis I.a.1.144 dadurch unterscheiden, daß R¹ und R³ für Chlor und R² für CF₃ stehen.

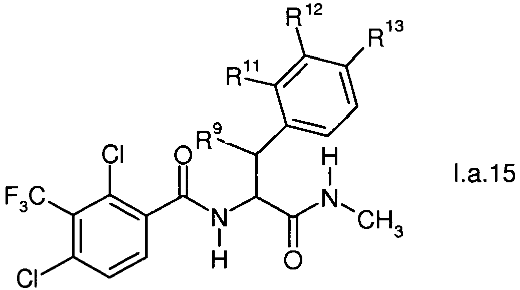

Ebenso außerordentlich bevorzugt sind die Verbindungen der Formel I.a.16, insbesondere die Verbindungen der Formel I.a.16.1 bis I.a.16.144, die sich von den entsprechenden Verbindungen der Formel I.a.1.1 bis I.a.1.144 dadurch unterscheiden, daß R¹ für CF₃ steht.

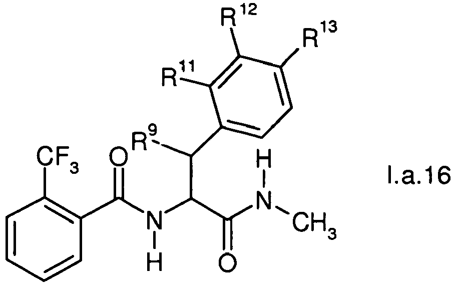

Ebenso außerordentlich bevorzugt sind die Verbindungen der Formel La.17, insbesondere die Verbindungen der Formel I.a.17.1 bis I.a.17.144, die sich von den entsprechenden Verbindungen der Formel I.a.1.1 bis I.a.1.144 dadurch unterscheiden, daß R¹ für CF₃ und R³ für Fluor steht.

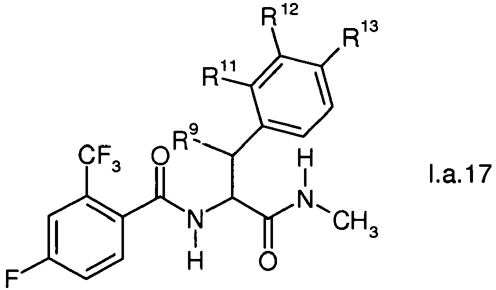

Ebenso außerordentlich bevorzugt sind die Verbindungen der Formel I.a.18, insbesondere die Verbindungen der Formel I.a.18.1 bis I.a.18.144, die sich von den entsprechenden Verbindungen der Formel I.a.1.1 bis I.a.1.144 dadurch unterscheiden, daß R¹ für CF₃ und R³ für Chlor steht.

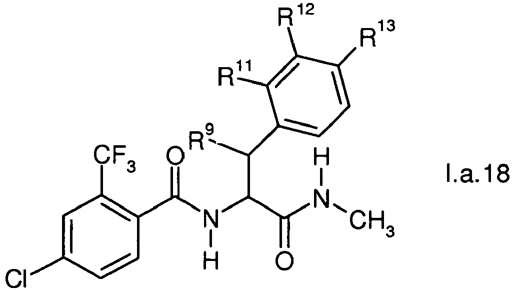

Ebenso außerordentlich bevorzugt sind die Verbindungen der Formel I.a.19, insbesondere die Verbindungen der Formel I.a.19.1 bis I.a.19.144, die sich von den entsprechenden Verbindungen der Formel I.a.1.1 bis I.a.1.144 dadurch unterscheiden, daß R¹ für CF₃ und R² für Fluor steht.

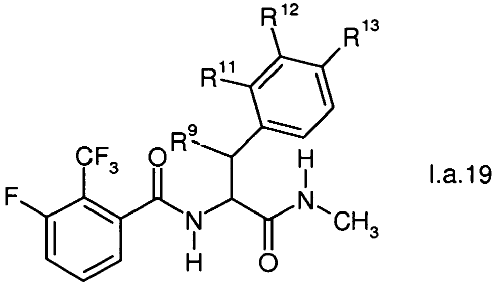

Ebenso außerordentlich bevorzugt sind die Verbindungen der Formel I.a.20, insbesondere die Verbindungen der Formel I.a.20.1 bis I.a.20.144, die sich von den entsprechenden Verbindungen der Formel I.a.1.1 bis I.a.1.144 dadurch unterscheiden, daß R¹ für CF₃ und R² sowie R³ für Fluor stehen.

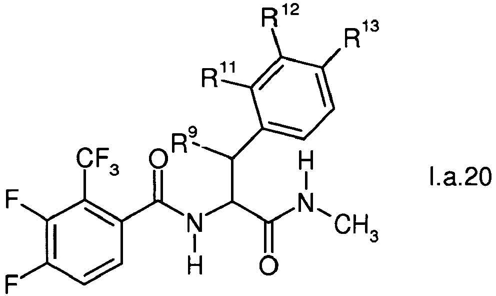

Ebenso außerordentlich bevorzugt sind die Verbindungen der Formel I.a.21, insbesondere die Verbindungen der Formel I.a.21.1 bis I.a.21.144, die sich von den entsprechenden Verbindungen der Formel I.a.1.1 bis I.a.1.144 dadurch unterscheiden, daß R¹ für CF₃, R² für Fluor und R³ für Chlor steht.

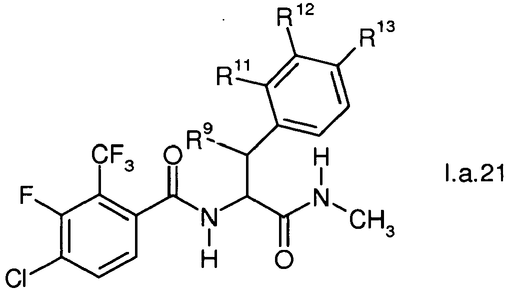

Ebenso außerordentlich bevorzugt sind die Verbindungen der Formel I.a.22, insbesondere die Verbindungen der Formel I.a.22.1 bis I.a.22.144, die sich von den entsprechenden Verbindungen der Formel I.a.1.1 bis I.a.1.144 dadurch unterscheiden, daß R¹ für CF₃ und R² für Chlor steht.

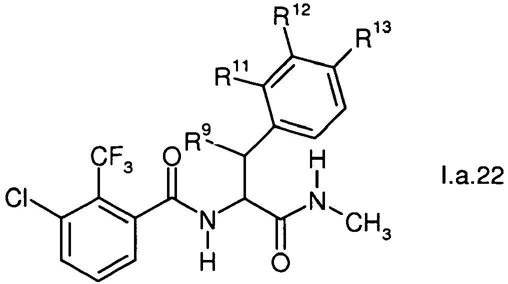

Ebenso außerordentlich bevorzugt sind die Verbindungen der Formel I.a.23, insbesondere die Verbindungen der Formel I.a.23.1 bis I.a.23.144, die sich von den entsprechenden Verbindungen der Formel I.a.1.1 bis I.a.1.144 dadurch unterscheiden, daß R¹ für CF₃, R² für Chlor und R³ für Fluor steht.

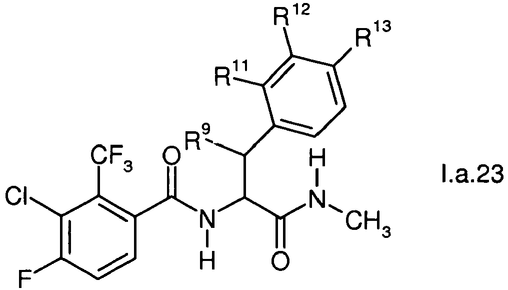

Ebenso außerordentlich bevorzugt sind die Verbindungen der Formel I.a.24, insbesondere die Verbindungen der Formel I.a.24.1 bis I.a.24.144, die sich von den entsprechenden Verbindungen der Formel I.a.1.1 bis I.a.1.144 dadurch unterscheiden, daß R¹ für CF₃ und R² sowie R³ für Chlor stehen.

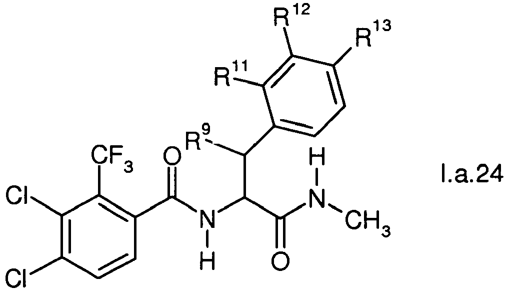

Ebenso außerordentlich bevorzugt sind die Verbindungen der Formel I.a.25, insbesondere die Verbindungen der Formel I.a.25.1 bis I.a.25.144, die sich von den entsprechenden Verbindungen der Formel I.a.1.1 bis I.a.1.144 dadurch unterscheiden, daß R¹ und R² für CF₃ stehen.

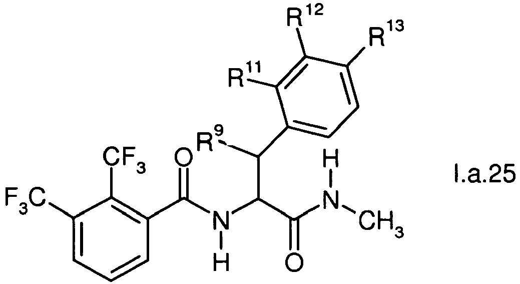

Ebenso außerordentlich bevorzugt sind die Verbindungen der Formel I.a.26, insbesondere die Verbindungen der Formel I.a.26.1 bis I.a.26.144, die sich von den entsprechenden Verbindungen der Formel I.a.1.1 bis I.a.1.144 dadurch unterscheiden, daß R¹ und R² für CF₃ und R³ für Fluor stehen.

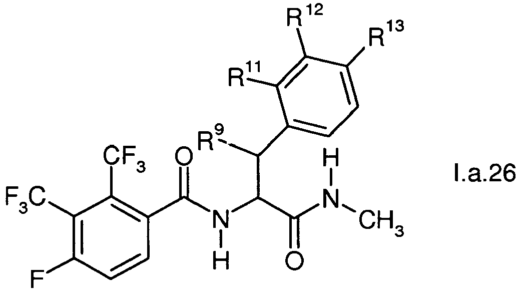

Ebenso außerordentlich bevorzugt sind die Verbindungen der Formel I.a.27, insbesondere die Verbindungen der Formel I.a.27.1 bis I.a.27.144, die sich von den entsprechenden Verbindungen der Formel I.a.1.1 bis I.a.1.144 dadurch unterscheiden, daß R¹ und R² für CF₃ und R³ für Chlor stehen.

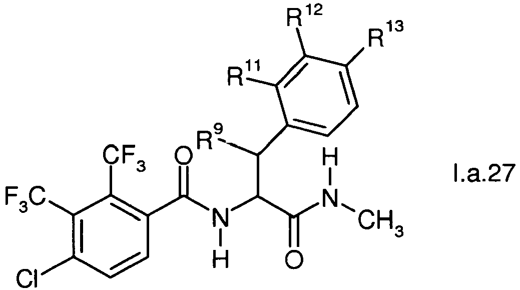

Ebenso außerordentlich bevorzugt sind die Verbindungen der Formel I.a.28, insbesondere die Verbindungen der Formel I.a.28.1 bis I.a.28.144, die sich von den entsprechenden Verbindungen der Formel I.a.1.1 bis I.a.1.144 dadurch unterscheiden, daß R¹ für CF₃ und R⁴ für Fluor stehen.

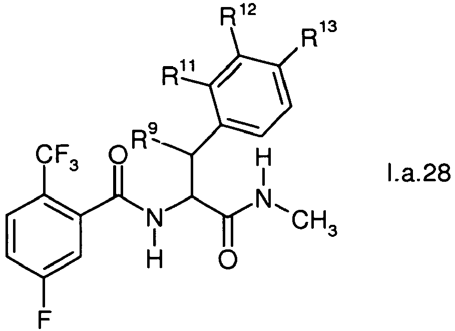

Ebenso außerordentlich bevorzugt sind die Verbindungen der Formel I.a.29, insbesondere die Verbindungen der Formel I.a.29.1 bis I.a.29.144, die sich von den entsprechenden Verbindungen der Formel I.a.1.1 bis I.a.1.144 dadurch unterscheiden, daß R¹ für CF₃ und R³ sowie R⁴ für Fluor stehen.

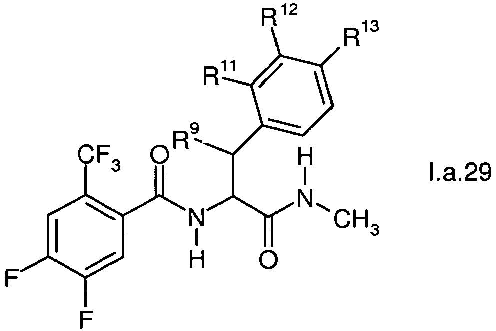

Die benzoylsubstituierten Phenylalanin-Amide der Formel I sind auf verschiedene Art und Weise erhältlich, beispielsweise nach folgenden Verfahren:

### Verfahren A

Ein Phenylalanin der Formel V wird zunächst mit Benzoesäuren bzw. Benzoesäurederivaten der Formel IV zu dem entsprechenden Benzoylderivat der Formel III umgesetzt, welches anschließend mit einem Amin der Formel II zu dem gewünschten benzoylsubstituierten Phenylalanin-Amid der Formel I reagiert:

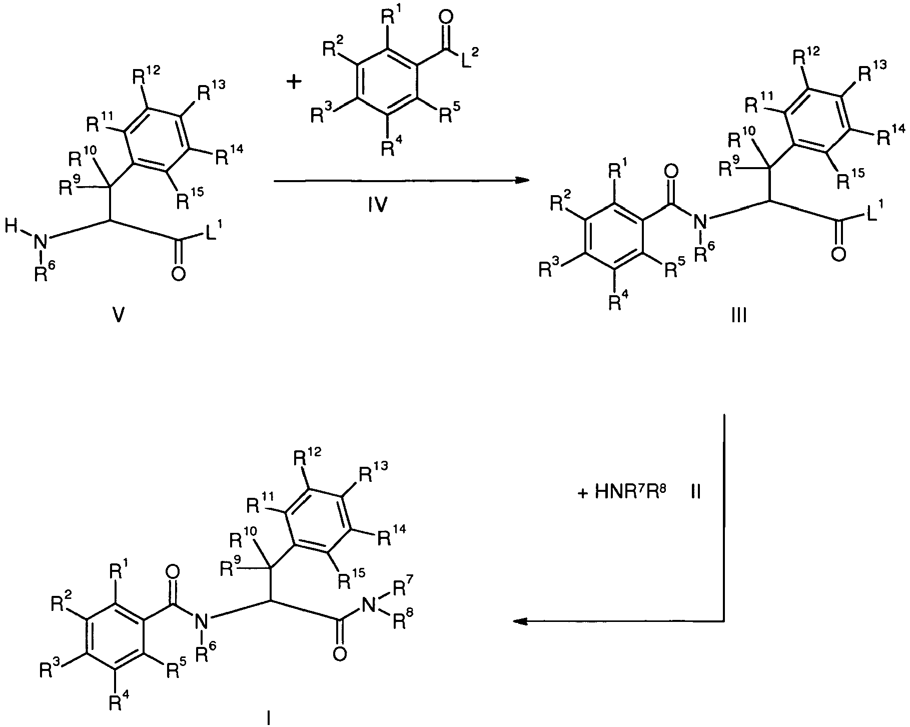

L¹ steht für eine nucleophil verdrängbare Abgangsgruppe, z.B. Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Phenylalanine der Formel V mit Benzoesäure(derivate)n der Formel IV, wobei L² für Hydroxy steht, zu Benzoylderivaten der Formel III erfolgt in Gegenwart eines Aktivierungsreagenz und einer Base üblicherweise bei Temperaturen von 0 °C bis zum Siedepunkt des Reaktionsgemisches, vorzugsweise 0°C bis 110°C, besonders bevorzugt bei Raumtemperatur, in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41 (3),593-595(2002); Clark, J. E. et al., Sythesis (10),891-894 (1991)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z. T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Umsetzung der Phenylalanine der Formel V mit Benzoesäuren bzw. Benzoesäurederivaten der Formel IV, wobei L² für Halogen oder C₁-C₆-Alkoxy steht, zu Benzoylderivaten der Formel III erfolgt in Gegenwart einer Base üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41 (3),593-595(2002); Clark, J. E. et al., Sythesis (10),891-894 (1991)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Aufarbeitung und Isolierung der Produkte kann in an sich bekannter Weise erfolgen.

Natürlich können auch in analoger Weise zunächst die Phenylalanine der Formel V mit Aminen der Formel II zu den entsprechenden Amiden umgesetzt werden, welche dann mit Benzoesäure(derivate)n der Formel IV zu den gewünschten benzoylsubstituierten Phenylalanin-Amiden der Formel I reagieren.

Die für die Herstellung der Benzoylderivate der Formel III benötigten Phenylalanine der Formel V mit L¹ = Hydroxy sind, auch in enantiomeren- und diastereomerenreiner Form, in der Literatur bekannt oder können gemäß der zitierten Literatur hergestellt werden:
R⁹ = OR¹⁶:
   - durch Kondensation von Glycinenolat-Equivalenten mit Benzaldehyden
      (Hvidt, T. et al., Tetrahedron Lett. 27 (33), 3807-3810 (1986) ; Saeed, A. et al., Tetrahedron 48 (12), 2507-2514 (1992); Kikuchi, J. et al., Chem. Lett. (3), 553-556 (1993) ; Soloshonok, V. A. et al., Tetrahedron Lett. 35 (17), 2713-2716 (1994) ; Soloshonok, V. A.; et al..; Tetrahedron 52 (1), 245-254 (1996); Rozenberg, V. et al., Angew. Chem. 106 (1), 106-108 (1994); US 4605759; Alker, D. et al., Tetrahedron 54 (22), 6089-6098 (1998) ; Shengde, W. et al., Synth. Commun. 16 (12), 1479 (1986); JP 2001046076; Herbert, R. B. et al., Can. J. Chem. 72 (1), 114-117 (1994)) ;
   - durch Spaltung von 2-N-Phtaloyl-3-Hydroxy-Phenylalaninen
      (Hutton, C. A., Org. Lett. 1 (2), 295-297(1999));
   - durch oxidative Aminohydroxylierung und anschließende Entschützung von Zimtsäurederivaten
      (Kim, I. H. et al., Tetrahedron Lett. 42 (48), 8401-8403 (2001);
   - durch Spaltung von substituierten Oxazolidinen
      (Wu, S. D. et al., Sythetic Commun. 16 (12), 1479-1484 (1986));
   - durch Spaltung von substituierten Oxazolinen
      (Soloshonok, V. A.; et al..; Tetrahedron 52 (1), 245-254 (1996); Lown, J. W. et al., Can. J. Chem. 51, 856 (1973));
   - durch Spaltung von substituierten 2-Oxazolidinonen
      (Jung, M. E. et al., Tetrahedron Lett. 30 (48), 6637-6640 (1989));
   - durch Spaltung von substituierten 5-Oxazolidinonen
      (Blaser, D. et al., Liebigs Ann. Chem. (10), 1067-1078 (1991));
   - durch Hydrolyse von Phenylserin-Nitril-Derivaten
      (Iriuchijima, S. et al., J. Am. Chem. Soc. 96, 4280 (1974))
   - durch Spaltung von substituierten Imidazolin-4-onen
      (Davis, C et al., J. Chem. Soc. 3479 (1951))

Die für die Herstellung der Benzoylderivate der Formel III benötigten Phenylalanine der Formel V mit L¹ = C₁-C₆-Alkoxy sind, auch in enantiomeren- und diastereomerenreiner Form, in der Literatur bekannt oder können gemäß der zitierten Literatur hergestellt werden:
R⁹ = OR¹⁶:
   - durch Kondensation von Glycinenolat-Equivalenten mit Aldehyden:
      Nicolaou, K. C. et al., J. Am. Chem. Soc. 124 (35), 10451-10455 (2002) ; Carrara, G. et al., Gazz. Chim. Ital. 82, 325 (1952); Fuganti, C. et al., J. Org. Chem. 51 (7), 1126-1128 (1986) ; Boger, D. L. et al., J. Org. Chem. 62 (14), 4721-4736 (1997); Honig, H. et al., Tetrahedron (46), 3841 (1990); Kanemasa, S. et al., Tetrahedron Lett. 34 (4), 677-680 (1993) ; US 4873359)
   - durch Spaltung von Dihydropyrazinen
      (Li, Y. Q. et al., Tetrahedron Lett. 40 (51), 9097-9100 (1999); Beulshausen, T. et al., Liebigs Ann. Chem. (11), 1207-1209 (1991))
   - durch Reduktion von N-Amino-Phenylserin-Derivaten
      ( Poupardin, O. et al., Tetrahedron Lett. 42 (8), 1523-1526 (2001))
   - durch Spaltung von N-Carbamoyl-Phenylserin-Derivaten
      (Park, H. et al., J. Org. Chem. 66 (21), 7223-7226 (2001) ; US 6057473; Kim, I. H. et al., Tetrahedron Lett. 42 (48), 8401-8403 (2001); Nicolaou, K. C. et al., Angew.N Chem. Int. Edit. 37 (19), 2714-2716 (1998))
   - durch Spaltung von substituierten Oxazolidinen
      (Zhou, C. Y. et al., Sythetic Commun. 17 (11), 1377-1382 (1987))
   - durch Reduktion von 2-Azido-3-Hydroxy-Phenylpropionsäure-Derivaten
      (Corey, E. J. et al., Tetrahedron Lett. 32 (25), 2857-2860 (1991))
   - durch Ringöffnung von Aziridinen mit Sauerstoff-Nukleophilen
      (Davis, F. A. et al., J. Org. Chem. 59 (12), 3243-3245 (1994))
   - durch Spaltung von substituierten 2-Oxazolidinonen
      (Jung, M. E. et al., Synlett 563-564 (1995))
   - durch Reduktion von 2-Hydroxyimino-3-Keto-Phenylpropionsäure-Derivaten
      (Inoue, H. et al., Chem. Phar. Bull. 41 (9), 1521-1523 (1993); Chang, Y.-T. et al., J. Am. Chem. Soc. 75, 89 (1953); US 4810817)
   - durch Hydrolyse von Phenylserin-Imino-Derivaten
      (Solladiecavallo, A. et al., Gazz. Chim. Ital. 126 (3), 173-178 (1996); Solladiecavallo, A. et al., Tetrahedron Lett. 39 (15), 2191-2194 (1998))
   - durch Spaltung von N-Acyl-Phenylserin-Derivaten
      (Girard, A. et al., Tetrahedron Lett. 37 (44), 7967-7970 (1996))
   - durch Reduktion von 2-Hydroxyimino-3-Hydroxy-Phenylpropionsäure-Derivaten
      (Boukhris, S. et al., Tetrahedron Lett. 40 (9), 1669-1672 (1999))
   - durch Spaltung von N-Benzyl-Phenylserin-Derivaten
      (Caddick, S.; Tetrahedron, 57 (30), 6615-6626 (2001))
   - durch Reduktion von 2-Diazo-3-Keto-Phenylpropionsäure-Derivaten
      (Looker, et al., J. Org. Chem. 22, 1233 (1957))
   - durch Spaltung von substituierten Imidazolidinonen
      (Davis, A. C.; et al., J. Chem. Soc. 3479 (1951))

Die für die Herstellung der Benzoylderivate der Formel III benötigten Benzoesäure(derivate) der Formel IV können käuflich erworben werden oder können analog zu literaturbekannten Vorschriften über eine Grignard-Redition aus dem ensprechenden Halogenid hergestellt werden [z.B. A. Mannschuk et al., Angew. Chem. 100, 299 (1988)].

Die Umsetzung der Benzoylderivate der Formel III mit L¹ = Hydroxy bzw. deren Salze mit einem Amin der Formel II zu den gewünschten benzoylsubstituierten Phenylalanin-Amiden der Formel I erfolgt in Gegenwart eines Aktivierungsreagenz und gegebenenfalls in Gegenwart einer Base üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel. [vgl. Perich, J. W., Johns, R. B., J. Org. Chem. 53 (17), 4103-4105 (1988); Somlai, C. et al., Synthesis (3), 285-287 (1992) ; Gupta, A. et al., J. Chem. Soc. Perkin Trans. 2, 1911 (1990); Guan et al., J. Comb. Chem. 2, 297 (2000)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein II in einem Überschuß bezogen auf III einzusetzen.

Die Aufarbeitung und Isolierung der Produkte kann in an sich bekannter Weise erfolgen.

Die Umsetzung der Benzoylderivate der Formel III mit L¹ = C₁-C₆-Alkoxy mit einem Amin der Formel II zu den gewünschten benzoylsubstituierten Phenylalanin-Amiden der Formel I erfolgt üblicherweise bei Temperaturen von 0 °C bis zum Siedepunkt des Reaktionsgemisches, vorzugsweise 0°C bis 100°C, besonders bevorzugt bei Raumtemperatur in einem inerten organischen Lösungsmittel gegebenenfalls in Gegenwart einer Base [vgl. Kawahata, N. H. et al., Tetrahedron Lett. 43 (40), 7221-7223 (2002); Takahashi, K. et al., J. Org. Chem. 50 (18), 3414-3415 (1985); Lee, Y. et al., J. Am. Chem. Soc. 121 (36), 8407-8408 (1999)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Die Umsetzung kann gegebenenfalls in Gegenwart einer Base erfolgen. Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, II in einem Überschuß bezogen auf III einzusetzen.

Die Aufarbeitung und Isolierung der Produkte kann in an sich bekannter Weise erfolgen.

Die für die Herstellung der benzoylsubstituierten Phenylalanin-Amide der Formel I benötigten Amine der Formel II können käuflich erworben werden.

### Verfahren B

Benzoylderivate der Formel III mit R⁹ = Hydroxy können auch erhalten werden, indem acylierte Glycin-Derivate der Formel VIII, wobei die Acylgruppe eine abspaltbare Schutzgruppe wie Benzyloxycarbonyl (vgl. VIIIa mit ∑ = Benzyl) oder tert.-Butyloxycarbonyl (vgl. VIIIa mit ∑ = tert-Butyl) sein kann, mit Heterocyclylcarbonyl-Verbindungenen VII zu entsprechenden Aldolprodukten VI kondensiert wird. Anschließend wird die Schutzgruppe abgespalten und die so entstandenen Phenylalanine der Formel V mit R⁹ = Hydroxy mit Benzoesäure(derivate)n der Formel IV acyliert.

Anlog kann auch ein acyliertes Glycin-Derivat der Formel VIII, wobei die Acylgruppe ein substituierter Benzoylrest (vgl. VIIIb) ist, unter Baseneinfluß mit einer Heterocyclylcarbonyl-Verbindung VII zum Benzoylderivat III mit R⁹ = Hydroxy umgesetzt werden:

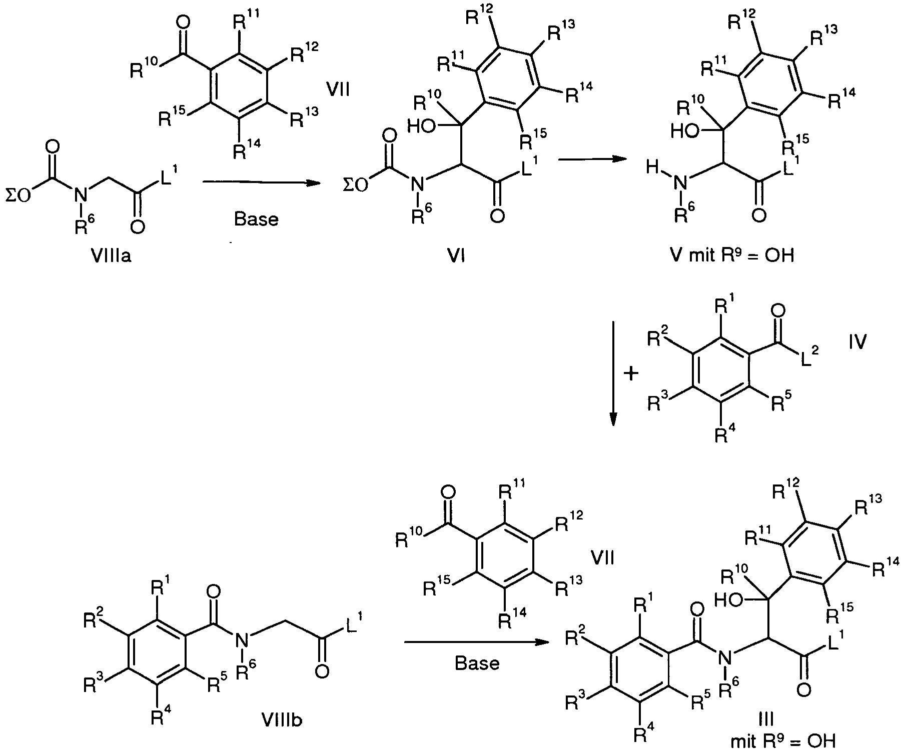

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Glycinderivate VIII mit Heterocyclyl-Verbindungen VII zum entsprechenden Aldolprodukt VI bzw. Benzoylderivat III mit R⁹ = Hydroxy erfolgt üblicherweise bei Temperaturen von -100°C bis zum Siedepunkt der Reaktionsmischung, bevorzugt - 80°C bis 20°C, insbesondere bevorzugt -80°C bis -20°C, in einem inerten organischen Lösungsmittel in Gegenwart einer Base [vgl. J.-F. Rousseau et al., J. Org. Chem. 63, 2731-2737 (1998)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calciumhydrid, Alkalimetallazide wie Lithiumhexamethyldisilazid, metallorganische Verbindungen, insbesondere Alkalimetallalkyle wie Methyllithium, Butyllithium und Phenyllithium, sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Lithiumhexamethyldisilazid und Lithiumdiisopropylamid.

Die Basen werden im allgemeinen in äquimolaren Mengen eingesetzt, sie können aber auch katalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder die Heterocyclylcarbonyl-Verbindungen VII in einem Überschuß bezogen auf die Glycinderivate VIII einzusetzen.

Die Aufarbeitung und Isolierung der Produkte kann in an sich bekannter Weise erfolgen.

Die für die Herstellung der Verbindungen I benötigten Glycinderivate der Formel VIII können käuflich erworben werden, sind in der Literatur bekannt [z.B. H. Pessoa-Mahana et al., Synth. Comm. 32, 1437 (2002] oder können gemäß der zitierten Literatur hergestellt werden.

Die Abspaltung der Schutzgruppe zu Phenylalaninen der Formel V mit R⁹ = Hydroxy erfolgt nach literaturbekannten Methoden[vgl. J.-F. Rousseau et al., J. Org. Chem. 63, 2731-2737 (1998)); J. M. Andres, Tetrahedron 56, 1523 (2000)];
im Fall von ∑ = Benzyl durch Hydrogenolyse, bevorzugt durch Wasserstoff und Pd/C in Methanol;im Fall von ∑ = tert.-Butyl durch Säure, bevorzugt Salzsäure in Dioxan.

Die Umsetzung der Phenylalanine V mit R⁹= Hydroxy mit Benzoesäure(derivate)n IV zu Benzoylderivaten III mit R⁹ = Hydroxy erfolgt üblicherweise analog der unter Verfahren A genannten Umsetzung der Phenylalanine der Formel V mit Benzoesäure(derivate)n der Formel IV zu Benzoylderivaten III.

Die Benzoylderivate der Formel III mit R⁹ = Hydroxy lassen sich anschließend mit Aminen der Formel II analog zu Verfahren A zu den gewünschten benzoylsubstituierten Phenylalanin-Amiden der Formel I mit R⁹ = Hydroxy umsetzen, welche dann mit Verbindungen der Formel IX zu benzoylsubstituierten Phenylalanin-Amiden der Formel I mit R⁹ = OR¹⁶ derivatisiert werden können [vgl. z.B. Yokokawa, F. et al., Tetrahedron Lett. 42 (34), 5903-5908 (2001); Arrault, A. et al., Tetrahedron Lett. 43( 22), 4041-4044 (2002)].

Ebenso können die Benzoylderivate der Formel III mit R⁹ = Hydroxy zunächst mit Verbindungen der Formel IX zu weiteren Benzoylderivaten der Formel III derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel II zu den gewünschten benzoylsubstituierten Phenylalanin-Amiden der Formel I mit R⁹ = OR¹⁶ umgesetzt werden:

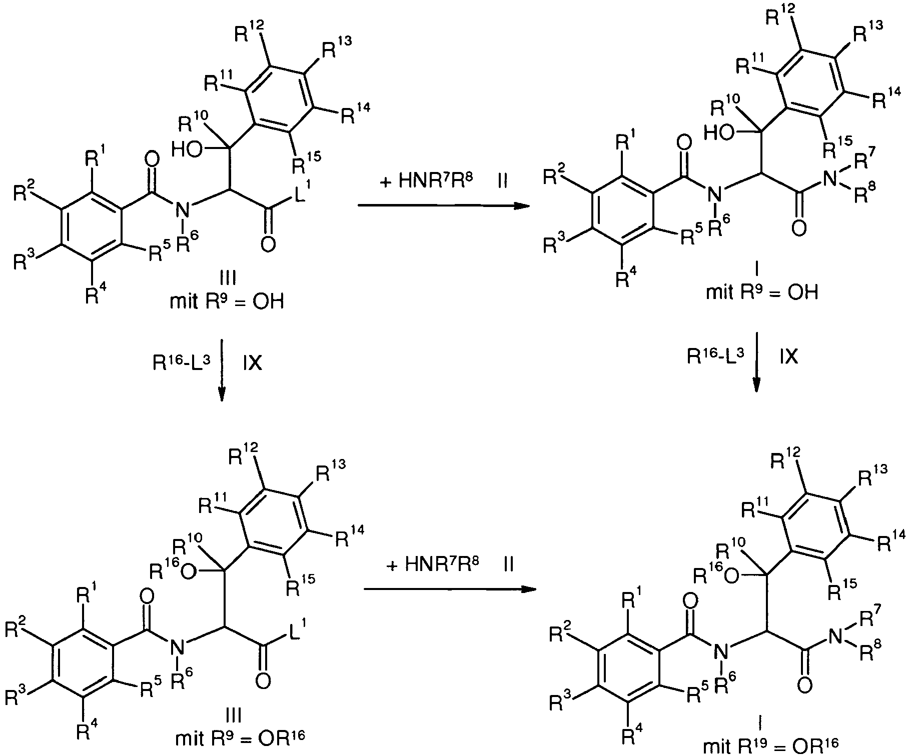

L¹ steht für eine nucleophil verdrängbare Abgangsgruppe, z.B. für Hydroxy oder C₁-C₆-Alkoxy.

L³ steht für eine nucleophil verdrängbare Abgangsgruppe, z.B. für Halogen, Hydroxy, oder C₁-C₆-Alkoxy.

Die Umsetzung der Benzoylderivate der Formel III mit R⁹ = Hydroxy bzw. OR¹⁶ mit Aminen der Formel II zu benzoylsubstituierten Phenylalanin-Amiden der Formel I mit R⁹ = Hydroxy bzw. OR¹⁶ erfolgt üblicherweise analog der unter Verfahren A geschilderten Umsetzung der Benzoylderivate der Formel III mit Aminen der Formel II.

Die Umsetzung der Benzoylderivate der Formel II mit R⁹ = Hydroxy bzw. der benzoylsubstituierten Phenylalanin-Amide der Formel I mit R⁹ = Hydroxy mit Verbindungen der Formel IX zu Benzoylderivaten der Formel III mit R⁹ = OR¹⁶ bzw. benzoylsubstituierten Phenylalanin-Amiden der Formel I mit R⁹ = OR¹⁶ erfolgt üblicherweise bei Temperaturen von 0°C bis 100°C, vorzugsweise 10°C bis 50°C, in einem inerten organischen Lösungsmittel in Gegenwart einer Base [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Dichlormethan, tert.-Butylmethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriumhydrid und Triethylamin.

Die Basen werden im allgemeinen in äquimolaren Mengen eingesetzt, sie können aber auchkatalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder IX in einem Überschuß bezogen auf III bzw. I einzusetzen.

Die Aufarbeitung und Isolierung der Produkte kann in an sich bekannter Weise erfolgen.

Die benötigten Verbindungen der Formel VIII können käuflich erworben werden.

### Verfahren C

Benzoylderivate der Formel III mit R⁹ = Hydroxy können auch erhalten werden, indem Aminomalonyl-Verbindungen der Formel XI zunächst mit Benzoesäure(derivate)n der Formel IV zu entsprechenden N-Acyl-Aminomalonyl-Verbindungen der Formel X acyliert werden und anschließend mit einer Heterocyclylcarbonyl-Verbindung der Formel VII unter Decarboxylierung kondensiert werden:

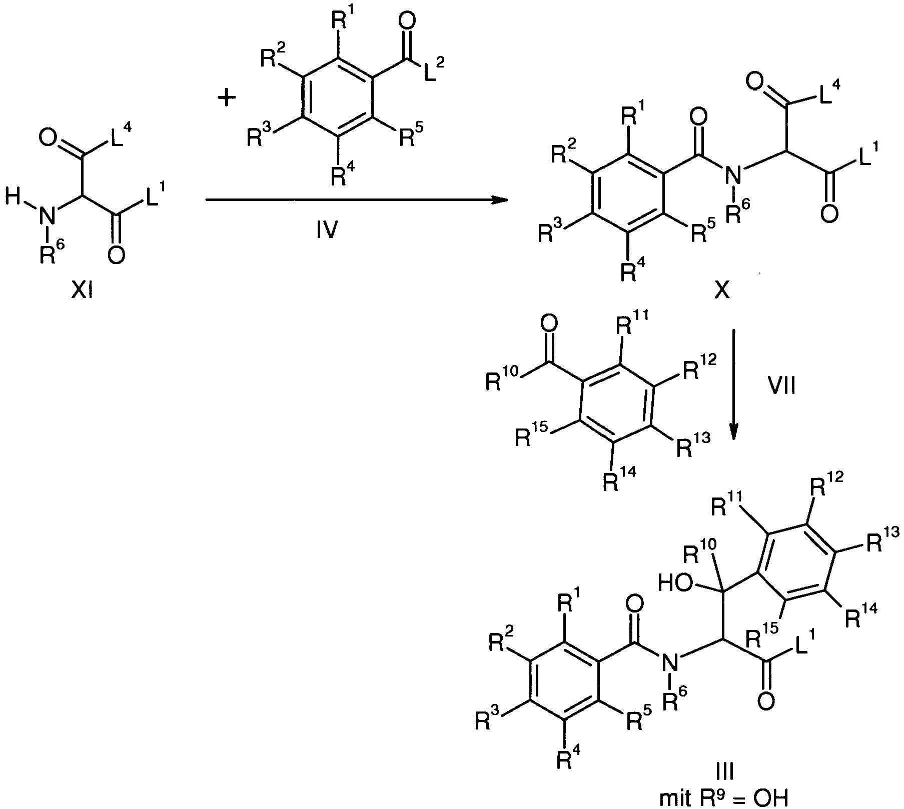

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

L⁴ steht für eine nucleophil verdrängbare Abgangsgruppe, z.B. für Hydroxy oder C₁-C₆-Alkoxy.

Die Acylierung der Aminomalonyl-Verbindungen der Formel XI mit Benzoesäure(derivate)n der Formel IV zu entsprechenden N-Acyl-Aminomalonyl-Verbindungen der Formel X erfolgt üblicherweise analog der unter Verfahren A genannten Umsetzung der Phenylalanine der Formel V mit Benzoesäure(derivate)n der Formel IV zu den entsprechenden Benzoylderivaten der Formel III.

Die Umsetzung der N-Acyl-Aminomalonyl-Verbindungen der Formel X mit Heterocyclylcarbonylverbindungen der Formel VII zu Benzoylderivaten der Formel III mit R⁹ = Hydroxy erfolgt üblicherweise bei Temperaturen von 0°C bis 100°C, vorzugsweise 10°C bis 50°C, in einem inerten organischen Lösungsmittel in Gegenwart einer Base [vgl. z.B. US 4904674; Hellmann, H. et al., Liebigs Ann. Chem. 631, 175-179 (1960)]

Falls L⁴ bei den N-Acyl-Aminomalonyl-Verbindungen der Formel X für C₁-C₆-Akoxy steht, ist es von Vorteil, L⁴ zunächst durch Esterverseifung [z.B. Hellmann, H. et al., Liebigs Ann. Chem. 631, 175-179 (1960)] in eine Hydroxygruppe zu überführen.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid,besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Diisopropylethylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, die Base in einem Überschuß bezogen auf X einzusetzen.

Die Aufarbeitung und Isolierung der Produkte kann in an sich bekannter Weise erfolgen.

Die so erhaltenen Benzoylderivate der Formel III mit R⁹ = Hydroxy können anschließend gemäß den voranstehend genannten Verfahren A bzw. B zu den gewünschten benzoylsubstituierten Phenylalanin-Amiden der Formel I mit R⁹ = OR¹⁶ umgesetzt werden.

Die benötigten Aminomalonyl-Verbindungen der Formel XI können käuflich erworben werden bzw. sind in der Literatur bekannt [z.B. US 4904674; Hellmann, H. et al., Liebigs Ann. Chem. 631, 175-179 (1960)] oder können gemäß der zitierten Literatur hergestellt werden.

Die benötigten heteroyclischen Verbindungen der Formel VII können käuflich erworben werden.

### Verfahren D

Benzoylderivate der Formel III mit R⁹ = Hydroxy und R¹⁰ = Wasserstoff können auch erhalten werden, indem Ketoverbindungen der Formel XIII zunächst mit Benzoesäure(derivate)n der Formel IV zu entsprechenden N-Acyl-Ketoverbindungen der Formel XII acyliert werden und anschließend die Ketogruppe reduziert wird [Girard A, Tetrahedron Lett. 37(44),7967-7970(1996); Nojori R., J. Am. Chem. Soc. 111(25),9134-9135(1989); Schmidt U., Synthesis (12),1248-1254 (1992); Bolhofer, A.; J. Am. Chem. Soc. 75, 4469 (1953)]:

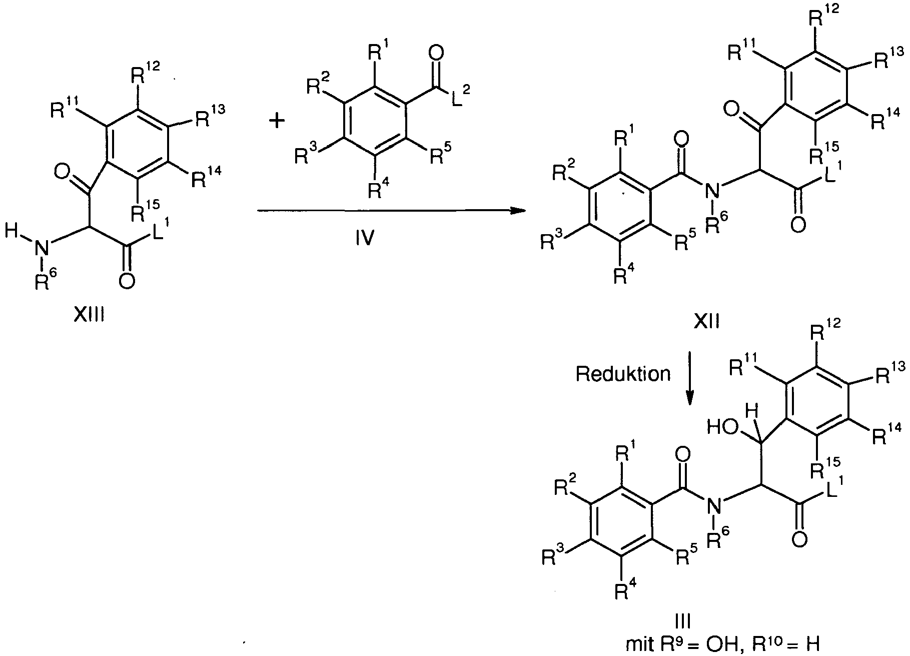

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

Die Acylierung der Ketoverbindungen der Formel XIII mit Benzoesäure(derivate)n der Formel IV zu N-Acyl-Ketoverbindungen der Formel XII erfolgt üblicherweise analog der unter Verfahren A genannten Umsetzung der Phenylalanine der Formel V mit Benzoesäure(derivate)n der Formel IV zu den entsprechenden Benzoylderivaten der Formel III.

Die für die Herstellung der Benzoylderivate der Formel III mit R⁹ = Hydroxy und R¹⁰ = Wasserstoff benötigten Ketoverbindungen der Formel XIII sind in der Literatur bekannt [WO 02/083111; Boto, A. et al., Tetrahedron Letters 39 (44), 8167-8170 (1988); von Geldern, T. et al., J. of Med. Chem. 39(4), 957-967 (1996); Singh, J. et al., TetrahedronLetters 34 (2), 211-214 (1993); ES 2021557; Maeda, S: et al., Chem. & Pharm. Bull. 32 (7), 2536-2543 (1984); Ito, S. et al., J. of Biol. Chem. 256 (15), 7834-4783 (1981); Vinograd, L. et al., Zhurnal Organicheskoi Khimii 16 (12), 2594-2599 (1980); Castro, A. et al., J. Org. Chem. 35 (8), 2815-2816 (1970); JP 02-172956; Suzuki, M. et al., J. Org. Chem. 38 (20), 3571-3575 (1973) ; Suzuki, M. et al, Synthetic Communications 2 (4), 237-242 (1972)] oder können gemäß der zitierten Literatur hergestellt werden.

Die Reduktion der N-Acyl-Ketoverbindungen der Formel XII zu Benzoylderivaten der Formel III mit R⁹ = Hydroxy und R¹⁰ = Wasserstoff erfolgt üblicherweise bei Temperaturen von 0°C bis 100°C, vorzugsweise 20°C bis 80°C, in einem inerten organischen Lösungsmittel in Gegenwart eines Reduktionsmittels.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Toluol, Methylenchlorid oder tert.-Butylmethylether.

Es können auch Gemische der genannten Lösungsmittel verwendet werden.

Als Reduktionsmittel eignen sich z.B. Natriumborhydrid, Zinkborhydrid, Natriumcyanoborhydrid, Litium-triethylborhydrid (Superhydrid®), Litium-tri-sec.butylborhydrid (L-Selectrid®), Litiumaluminiumhydrid oder Boran [vgl. z.B. WO 00/20424; Marchi, C. et al., Tetrahedron 58 (28), 5699 (2002); Blank, S. et al., Liebigs Ann. Chem. (8), 889-896 (1993); Kuwano, R. et al., J. Org .Chem. 63 (10), 3499-3503 (1998); Clariana, J. et al., Tetrahedron 55 (23), 7331-7344 (1999)].

Weiterhin kann die Reduktion auch in Gegenwart von Wasserstoff und eines Katalysator erfolgen. Als Katalysatoren eignen sich z.B. [Ru(BINAP)Cl₂] oder Pd/C [vgl. Noyori, R. et al., J. Am. Chem. Soc. 111 (25), 9134-9135 (1989); Bolhofer, A. et al., J. Am. Chem. Soc. 75, 4469 (1953)].

Daneben kann die Reduktion auch Gegenwart eines Mikroorganismus erfolgen. Als Mikroorganismus eignet sich z.B. *Saccharomyces Rouxii* [vgl. Soukup, M. et al., Helv. Chim. Acta 70, 232 (1987)].

Die N-Acyl-Ketoverbindungen der Formel XII und das jeweilige Reduktionsmittel werden im allgemeinen in äquimolaren Mengen miteinander umgesetzt. Es kann vorteilhaft sein, das Reduktionsmittel in einem Überschuß bezogen auf XII einzusetzen.

Die Aufarbeitung und Isolierung der Produkte kann in an sich bekannter Weise erfolgen.

Die so erhaltenen Benzoylderivate der Formel III mit R⁹ = Hydroxy und R¹⁰ = Wasserstoff können anschließend gemäß den voranstehend genannten Verfahren A und B zu den gewünschten benzoylsubstituierten Phenylalanin-Amiden der Formel I mit R⁹ = OR¹⁶ umgesetzt werden.

### Benzoylderivate der Formel III

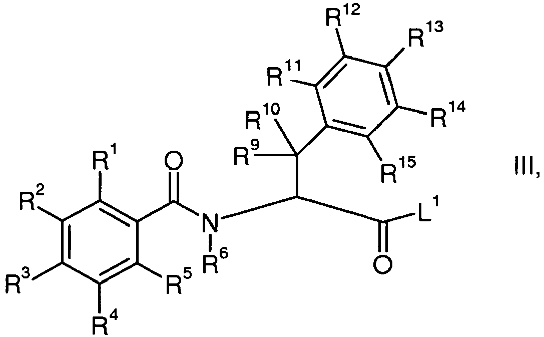

wobei R¹ bis R⁶ und R⁹ bis R¹⁵ die unter Anspruch 1 genannten Bedeutungen haben und L¹ für eine nucleophil verdrängbare Abgangsgruppe wie Hydroxy oder C₁-C₆-Alkoxy steht, sind ebenfalls ein Gegenstand der vorliegenden Erfindung.

Die besonders bevorzugten Ausführungsformen der Benzoylderivate der Formel III in Bezug auf die Variablen entsprechen denen der Reste R¹ bis R⁶ und R⁹ bis R¹⁵ der Formel I.

Besonders bevorzugt werden Benzoylderivate der Formel III, in denen
- R¹: Fluor, Chlor oder CF₃,
- R² und R³: unabhängig voneinander Wasserstoff, Fluor oder Chlor,
- R⁴, R⁵ und R⁶: Wasserstoff,
- R⁹: OR¹⁶;
- R¹⁰: Wasserstoff;
- R¹¹: Wasserstoff, Fluor oder CH₃;
- R¹²: Wasserstoff, Fluor oder Chlor;
- R¹³, R¹⁴ und R¹⁵: Wasserstoff; und
- R¹⁶: Wasserstoff, C₁-C₄-Alkylcarbonyl, C₁-C₄-Alkylaminocarbonyl, Di-(C₁-C₄-alkyl)-aminocarbonyl, Phenylaminocarbonyl, N-(C₁-C₄-alkyl)-N-(phenyl)-aminocarbonyl, SO₂CH₃, SO₂CF₃ oder SO₂(C₆H₅);
bedeuten.

### Beispiel 1

### (2S,3R)-Methyl-phenyl-carbaminsäure-2-(4-fluoro-2-trifluoromethyl-benzoylamino)-2-methylcarbamoyl-1-o-tolyl-ethyl-ester (Tab. 3, Nr. 3,34)

### 1.1) 2-Amino-3-oxo-3-o-tolyl-propionsäureethylester-hydrochlorid

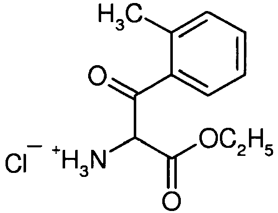

4.2 g (0.038 mol) Kalium-tert-butylat wurden unter Stickstoff in THF suspendiert. Es wurde auf -78°C gekühlt und 10.0 g (0.037 mol) N-(Diphenylmethylen)-glycinethylester gelöst in THF zugetropft. Nach 40 min bei -78 °C wurde die Lösung in einen gekühlten Tropftrichter (-78°C) überführt und zu einer auf -78°C gekühlten Lösung von 2-Methylbenzoylchlorid in THF getropft. Nach 1 h Rühren bei -78°C ließ man die Reaktionsmischung innerhalb von 2 h auf 0°C erwärmen. Es wurde mit 10%-iger salzsäure hydrolysiert und nachgerührt. Die Lösungsmittel wurden entfernt, der Rückstand in Wasser aufgenommen und mit MTBE gewaschen. Die Wasserphase wurde eingeengt, der Rückstand mit Methanol versetzt und abfiltriert. Nach Einengen des Filtrats erhielt man 6.2g der Titelverbindung als farbloses Öl.
¹H-NMR (DMSO): δ = 9.3 (br,3H, NH); 7.3-7.6 (m, 4H), 4.1 (m, 2H); 3.7 (m, 1 H); 2.40 (s, 3H); 0.95 (t, 3H).

### 1.2) 2-(4-Fluoro-2-trifluoromethyl-benzoylamino)-3-oxo-3-o-tolyl-propionsäureethylester

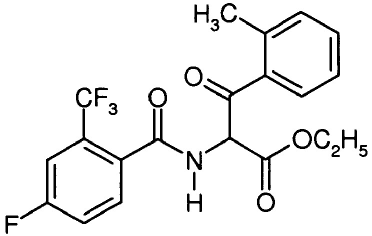

6.2 g (0.024mol) 2-Amino-3-oxo-3-o-tolyl-propionsäureethylester hydrochlorid wurden in Methylenchlorid gelöst und 9.7 g (0.096 mol) Triethylamin zugegeben. Hierzu wurden bei 0°C 5.4 g (0.024 mol) 4-Fluor-2-trifluormethylbenzoylchlorid gelöst in Methylenchlorid zugetropft. Es wurde 1 h bei Raumtemperatur (RT) gerührt und anschließend mit 5%iger Salzsäure versetzt. Die organische Phase wurde abgetrennt, gewaschen, getrocknet und das Lösungsmittel entfernt. Nach chromatographischer Reinigung (Kieselgelsäule, Cyclohexan/Essigsäureethylester) erhielt man 4.7g der Titelverbindung als farblose Kristalle.
¹H-NMR (DMSO): δ = 9.61 (d, 1 H); 7.3-7.9 (m, 7H); 6.18 (d, 1 H); 4.1-4.3 (m, 2H); 2.40 (s, 3H);1.15 (t, 3H).

### 1.3) (2S,3R)-2-(4-Fluoro-2-trifluoromethyl-benzoylamino)-3-hydroxy-3-o-tolyl-propion-säureethylester

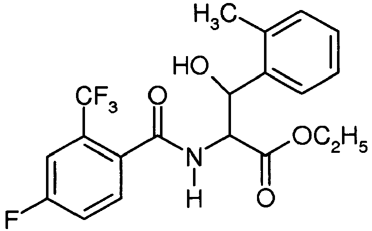

4.7 g (0.0114 mol) 2-(4-Fluoro-2-trifluoromethyl-benzoylamino)-3-oxo-3-o-tolyl-propionsäureethylester wurden in Methylenchlorid gelöst, die Lösung im Ultraschallbad entgast und 200 mg Katalysatormischung versetzt. Die Katalysatormischung wurde zuvor durch 1 h erhitzen von 78 mg Dichloro(P-Cymene)ruthenium(II)-Dimer (RuCl₂Cy)und 138 mg BINAP in Methylenchlorid und Ethanol auf 50°C und anschließendem Entfernen der Lösungsmittel hergestellt.

Die Lösung wurde unter 80 bar Wasserstoffdruck bei 50°C 90 h erhitzt. Nach Entfernen der Lösungsmittel und chromatographischer Reinigung (Kieselgelsäule, Cyclohexan/Essigsäureethylester) erhielt man 3.4 g der Titelverbindung als farblose Kristalle.
¹H-NMR (DMSO): δ = 8.95 (d, 1 H); 7.0-8.7 (m, 7H); 5.80 (d, 1 H); 5.40 (t, 1 H); 4.75 (dd, 1 H); 4.10 (m, 2H); 2.30 (s, 3H); 1.20 (t, 3H).

### 1.4) (2S,3R)-2-(4-Fluoro-2-trifluoromethyl-benzoylamino)-3-hydroxy-3-o-tolyl-propion-säure-N-methylamid

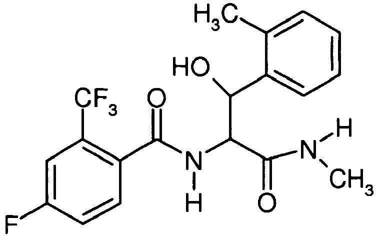

3.4 g (0.0082 mol) (2S,3R)-2-(4-Fluoro-2-trifluoromethyl-benzoylamino)-3-hydroxy-3-o-tolyl-propion-säureethylester wurden in Ethanol gelöst. Bei Raumtemperatur leitete man Methylamin-Gas ein. Nach 1.5h wurde für 1 h auf 30-35 °C erwärmt. Nach Entfernen der Lösungsmittel erhielt man 3.1g der Titelverbindung als farblose Kristalle.
¹H-NMR (DMSO): δ = 8.45 (d, 1 H); 7.0-7.7 (m, 7H); 5.70 (d, 1 H); 5.30 (t, 1 H); 4.65 (dd, 1 H); 2.65 (d, 3H); 2.40 (s, 3H); 1.10 (t, 3H).

### 1.5) (2S,3R)-2-(4-Fluoro-2-trifluoromethyl-benzoylamino)-3-(N-phenyl-N-methylaminocarbonyloxy)-3-o-tolyl-propionsäure-N-methylamid (Tab. 3, Nr. 3.34)

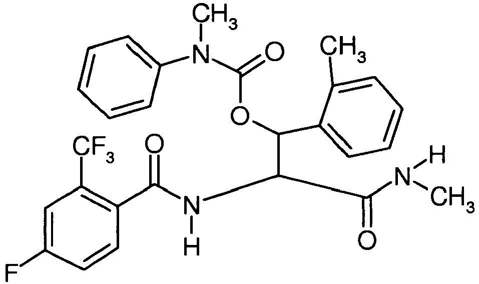

0.4 g (0.001 mol) (2S,3R)-2-(4-Fluoro-2-trifluoromethyl-benzoylamino)-3-hydroxy-3-o-tolyl-propion-säure-N-methylamid wurden in Methylenchlorid gelöst, 013 g (0.0013 mol) Triethylamin und eine Spatelspitze 4-Dimetylaminopyridin zugesetzt und 0.22g N-Phenyl-N-Methyl-Carbamoylchlorid in Methylenchlorid zugetropft. Die Suspension wurde 15 Stunden gerührt, mit 5% Salzsäure und NaHCO₃-Lösung extrahiert und getrocknet. Nach chromatographischer Reinigung (Kieselgelsäule, Cyclohexan/Essigsäureethylester) erhielt man 0.28g der Titelverbindung als farbloses Öl.
¹H-NMR (DMSO): δ = 8.8 (br, 1 H); 7.0-7.6 (m, 12H); 5.70 (d, 1 H); 5.30 (br, 1 H); 4.85 (dd, 1 H); 2.75 (d, 3H); 2.55 (d, 3H); 2.40 (s, 3H).

### Beispiel 3

### 3-Chloro-2-trifluormethyl-benzoesäure

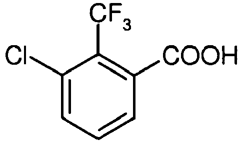

1,03 g (42,4 mmol) Magnesiumspäne wurden in THF gelöst. Man gab 2 Tropfen 1,2-Dibrommethan zu und rührte die Reditionsmischung nach Beginn der exothermen Reaktion bei 32-35°C unter Eiskühlung. Anschließend wurden 10,0 g (38,5 mmol) 1-Brom-3-chlor-2-trifluormethylbenzol in THF so zugetropft, dass die Temperatur 32°C nicht überstieg. Man rührte 30 min. nach, kühlte auf 0°C ab und leitete über 2 h Kohlendioxid ein. Anschließend wurde auf Raumtemperatur erwärmt und eine weitere Stunde CO₂ eingeleitet.

Man goß die Lösung auf eine Mischung von 1 M Salzsäure und Eis und extrahierte mit Methyl-tert-butylether. Die organische Phase wurde dann mit 1 M NaOH extrahiert, die wässrige Phase mit konz. Salzsäure angesäuert und mit Methylenchlorid extrahiert. Nach Trocknen und destillativer Entfernung des Lösungsmittels erhielt man 7,7 g (84 % der Theorie) der Titelverbindung als farblose Kristalle (Schmp. 110°C).

In den nachfolgenden Tabellen 2 und 3 sind neben den voranstehenden Verbindungen noch weitere Benzoylderivate der Formel III sowie benzoylsubstituierte Phenylalanin-Amide der Formel I aufgeführt, die in analoger Weise nach den voranstehend beschriebenen Verfahren hergestellt wurden oder herstellbar sind.

## Patentansprüche

1. Benzoylsubstituierte Phenylalanin-Amide der Formel I 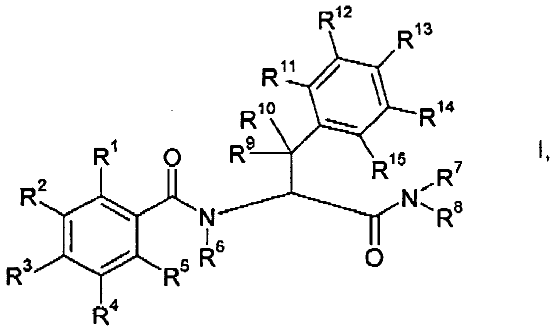 in der die Variablen die folgenden Bedeutungen haben:
R¹ Halogen, Cyano, C₁-C₆-Alkyl, C₁-C₆-Halogenalkyl, C₁-C₆-Halogenalkoxy, Nitro, Hydroxycarbonyl, C₁-C₆-Alkoxycarbonyl, C₁-C₆-Halogenalkylthio oder Phenyl;
R², R³, R⁴, R⁵ Wasserstoff, Halogen, Cyano, C₁-C₆-Alkyl, C₁-C₆-Halogenalkyl, C₁-C₆-Alkoxy, C₁-C₆-Halogenalkoxy, Nitro, Amino, C₁-C₆-Alkylamino, Di(C₁-C₆-alkyl)amino, C₁-C₆-Alkylthio oder C₁-C₆-Alkoxycarbonyl;
R⁶, R⁷ Wasserstoff, Hydroxy oder C₁-C₆-Alkoxy;
R⁸ C₁-C₆-Alkyl, C₁-C₄-Cyanoalkyl oder C₁-C₆-Halogenalkyl;
R⁹ OR¹⁶
R¹⁰ Wasserstoff oder C₁-C₆-Alkyl;
R¹¹, R¹² Wasserstoff, Halogen, Cyano, C₁-C₆-Alkyl, C₁-C₆-Halogenalkyl, Hydroxy, C₁-C₆-Alkoxy, C₁-C₆-Halogenalkoxy, Hydroxy, Nitro, Hydro-xy-C₁-C₄-alkyl, C₁-C₆-Alkoxy-C₁-C₄alkyl, Tri(C₁-C₆-alkyl)silyloxy-C₁-C₄-alkyl, C₁-C₄-Alkylthio, (Hydroxycarbonyl)-C₁-C₆-alkyl, (C₁-C₆-Alkoxycarbonyl)-C₁-C₆-alkyl, (Hydroxycarbonyl)-C₂-C₆-alkenyl, (C₁-C₆-Alkoxycarbonyl)-C₂-C₆-alkenyl, (Hydroxycarbonyl)-C₁-C₄-alkoxy, (C₁-C₄-Alkoxycarbonyl)-C₁-C₄-alkoxy, (C₁-C₄-Alkylcarbonyl)oxy-C₁-C₄-alkyl, Hydroxycarbonyl-C₁-C₄-alkoxy-C₁-C₄-alkyl, (C₁-C₄-Alkyl-sulfonyl)oxy-C₁-C₄-alkyl, C₁-C₄-Alkyl-O-C(O)-[C₁-C₄-alkyl-O]₃-C₁-C₄-alkyl, Carbamoyloxy-C₁-C₄-alkyl, (C₁-C₄-Alkylaminocarbonyl)oxy-C₁-C₄-alkyl, [Di(C₁-C₄-alkyl)aminocarbonyl]oxy-C₁-C₄-alkyl, [(C₁-C₄-Halogenalkylsulfonyl)aminocarbonyl]oxy-C₁-C₄-alkyl, Benzyloxy, wobei der Phenylring durch 1 bis 3 Reste aus der Gruppe Halogen und C₁-C₄-Alkyl substituiert sein kann,
Amino, C₁-C₄-Alkylamino, Di(C₁-C₄-alkyl)amino, (C₁-C₄-Alkyl-sulfonyl)amino, C₁-C₄-(Halogenalkylsulfonyl)amino, (C₁-C₄-Alkylcarbonyl)amino, Carbamoylamino, (C₁-C₄-Alkylamino)carbonylamino, [Di(C₁-C₄-alkyl)amino]-carbonylamino, [(C₁-C₄-Halogenalkylsulfonyl)-aminocarbonyl]amino, Phenyl oder Heterocyclyl, wobei der Phenyl-und der Heterocyclylrest der zwei letztgenannten Substituenten ein bis drei Reste aus folgender Gruppe tragen kann: Halogen, Nitro, C₁-C₄-Alkyl, C₁-C₄-Halogenalkyl, Hydroxycarbonyl und C₁-C₆-Alkoxycarbonyl;
R¹³, R¹⁴, R¹⁵ Wasserstoff, Halogen, Cyano, C₁-C₆-Alkyl, C₁-C₆-Halogenalkyl, C₁-C₆-Alkoxy, C₁-C₆-Halogenalkoxy, Nitro, Hydroxy, C₁-C₄-Alkyl-thio oder Benzyloxy;
R¹⁶ Wasserstoff, C₁-C₆-Alkyl, Tri(C₁-C₆-alkyl)silyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, C₁-C₆-Halogenalkylsulfonylaminocarbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N-(Di-C₁-C₆-alkylamino)-imino-C₁-C₆-alkyl,
wobei die genannten Alkyl-, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl. Aminocarbonyl, C₁-C₄-Alkylaminocarbonyl, Di-(C₁-C₄-alkyl)-aminocarbonyl oder C₁-C₄-Alkylcarbonyloxy;
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Heterocyclyl-C₁-C₆-alkyl, Heterocyclylcarbonyl, Heterocyclylcarbonyl-C₁-C₆-alkyl, Heterocyclyoxycarbonyl, Heterocyclylaminocarbonyl, Heterocyclylsulfonylaminocarbonyl, N-(C₁-C₆-Alkyl)-N-(heterocyclyl)-aminocarbonyl oder Heterocyclyl-C₁-C₆-alkylcarbonyl,
wobei der Phenyl- und der Heterocyclyl-Rest der 17 letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy
SO₂R¹⁷; -C(O)-[C₁-C₄-alkyl-O]₃-C₁-C₄-Alkyl; oder -C(O)-O-C₁-C₄-Alkyl-O-Phenyl, wobei der Phenylrest gegebenenfalls durch ein bis drei Reste aus der Gruppe Halogen und C₁-C₄-Alkyl substituiert sein kann;
R¹⁷ C₁-C₈-Alkyl, C₁-C₈-Halogenalkyl oder Phenyl,
wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

2. Benzoylsubstituierte Phenylalanin-Amide der Formel I gemäß Anspruch 1, wobei R¹ für Halogen oder C₁-C₆-Halogenalkyl steht.

3. Benzoylsubstituierte Phenylalanin-Amide der Formel I gemäß Anspruch 1 oder 2, wobei R² und R³ unabhängig voneinander für Wasserstoff, Halogen oder C₁-C₆-Halogenalkyl stehen.

4. Benzoylsubstituierte Phenylalanin-Amide der Formel I gemäß Ansprüchen 1 bis 3, wobei R⁴, R⁵, R⁶, R⁷, R¹⁰, R¹³, R¹⁴ und R¹⁵ für Wasserstoff stehen.

5. Verfahren zur Herstellung von benzoylsubstituierten Phenylalanin-Amiden der Formel I gemäß Anspruch 1, **dadurch gekennzeichnet dass**
Phenylalanine der Formel V 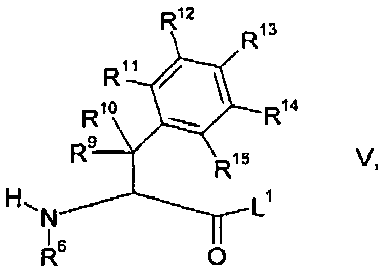 wobei R⁶ und R⁹ bis R¹⁵ die unter Anspruch 1 genannten Bedeutungen haben und L¹ für eine nucleophil verdrängbare Abgangsgruppe steht,
mit Benzoesäuren bzw. Benzoesäurederivaten der Formel IV 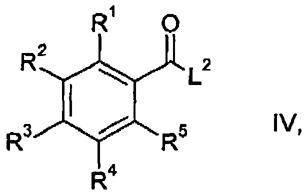 wobei R¹ bis R⁵ die unter Anspruch 1 genannten Bedeutungen haben und L² für eine nucleophil verdrängbare Abgangsgruppe steht,
zu entsprechenden Benzoylderivaten der Formel III 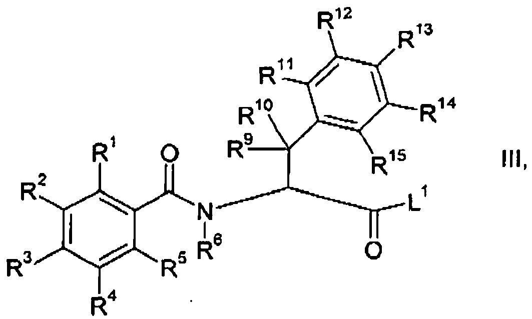 wobei R¹ bis R⁶ und R⁹ bis R¹⁵ die unter Anspruch 1 genannten Bedeutungen haben und L¹ für eine nucleophil verdrängbare Abgangsgruppe steht,
und anschließend die erhaltenen Benzoylderivaten der Formel III mit einem Amin der Formel II
NNR⁷R⁸ II,
wobei R⁷ und R⁸ die unter Anspruch 1 genannten Bedeutungen haben,
umgesetzt werden.

6. Verfahren zur Herstellung von benzoylsubstituierten Phenylalanin-Amiden der Formel **I** gemäß Anspruch 1, wobei R⁹ für Hydroxy und R¹⁰ für Wasserstoff stehen, **dadurch gekennzeichnet dass** Benzoylderivate der Formel III wobei R⁹ für Hydroxy und R¹⁰ für Wasserstoff stehen, durch Acylierung von Ketoverbindungen der Formel XIII 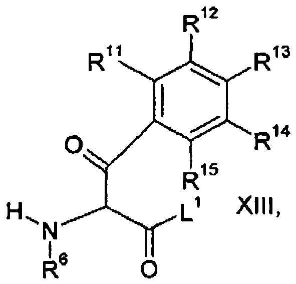 wobei R⁶ sowie R¹¹ bis R¹⁶ die unter Anspruch 1 genannten Bedeutungen haben und L¹ für eine nucleophil verdrängbare Abgangsgruppe steht,
mit Benzoesäure(derivate)n der Formel IV zu N-Acyl-Ketoverbindungen der Formel XII 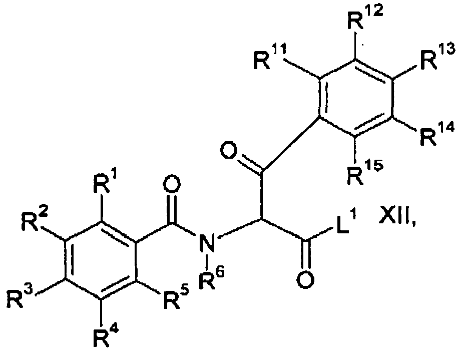 wobei R¹ bis R⁶ sowie R¹¹ bis R¹⁵ die unter Anspruch 1 genannten Bedeutungen haben und L¹ für eine nucleophil verdrängbare Abgangsgruppe steht, und anschließender Reduktion der Ketogruppe hergestellt werden.

7. Benzoylderivate der Formel III 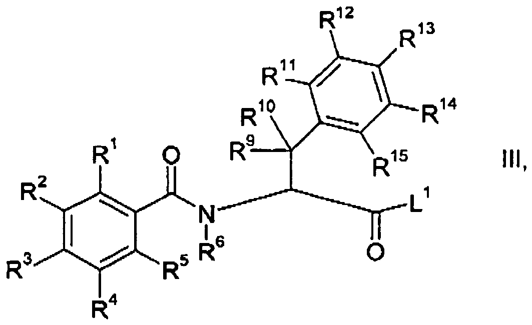 wobei R¹ bis R⁶ und R⁹ bis R¹⁵ die unter Anspruch 1 genannten Bedeutungen haben und L¹ für eine nucleophil verdrängbare Abgangsgruppe steht.

8. Mittel, enthaltend eine herbizid wirksame Menge mindestens eines benzoylsubstituierten Phenylalanin-Amids der Formel I oder eines landwirtschaftlich brauchbaren Salzes von I gemäß den Ansprüchen 1 bis 4 und für die Formulierung von Pflanzenschutzmitteln übliche Hilfsmittel.

9. Verfahren zur Herstellung von Mitteln gemäß Anspruch 8, **dadurch gekennzeichnet, daß** man eine herbizid wirksame Menge mindestens eines benzoylsubstituierten Phenylalanin-Amids der Formel I oder eines landwirtschaftlich brauchbaren Salzes von I gemäß den Ansprüchen 1 bis 4 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens eines benzoylsubstituierten Phenylalanin-Amids der Formel I oder eines landwirtschaftlich brauchbaren Salzes von I gemäß den Ansprüchen 1 bis 4 auf Pflanzen, deren Lebensraum und/oder auf Samen einwirken läßt.

11. Verwendung der benzoylsubstituierten Phenylalanin-Amide der Formel I gemäß den Ansprüchen 1 bis 4 und deren landwirtschaftlich brauchbaren Salze als Herbizide.

## Claims

1. A benzoyl-substituted phenylalanineamide of the formula I 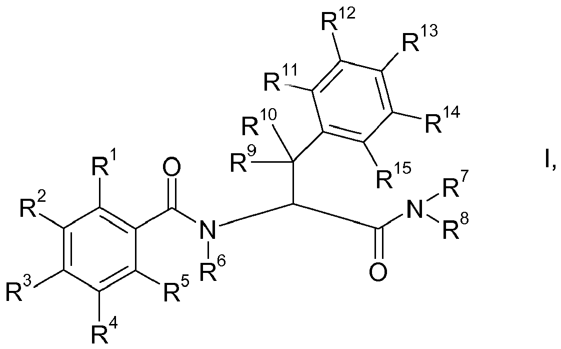 in which the variables are as defined below:
R¹ is halogen, cyano, C₁-C₆-alkyl, C₁-C₆-haloalkyl, C₁-C₆-haloalkoxy, nitro, hydroxycarbonyl, C₁-C₆-alkoxycarbonyl, C₁-C₆-haloalkylthio or phenyl;
R², R³, R⁴, R⁵ are hydrogen, halogen, cyano, C₁-C₆-alkyl, C₁-C₆-haloalkyl, C₁-C₆-alkoxy, C₁-C₆-haloalkoxy, nitro, amino, C₁-C₆-alkylamino, di(C₁-C₆-alkyl)amino, C₁-C₆-alkylthio or C₁-C₆-alkoxycarbonyl;
R⁷, R⁷ are hydrogen, hydroxyl or C₁-C₆-alkoxy;
R⁸ is C₁-C₆-alkyl, C₁-C₄-cyanoalkyl or C₁-C₆-haloalkyl;
R⁹ is OR¹⁶;
R¹⁰ is hydrogen or C₁-C₆-alkyl;
R¹¹, R¹² are hydrogen, halogen, cyano, C₁-C₆-alkyl, C₁-C₆-haloalkyl, hydroxyl, C₁-C₆-alkoxy, C₁-C₆-haloalkoxy, hydroxyl, nitro, hydroxy-C₁-C₄-alkyl, C₁-C₆-alkoxy-C₁-C₄-alkyl, tri(C₁-C₆-alkyl) silyloxy-C₁-C₄-alkyl, C₁-C₄-alkylthio, (hydroxycarbonyl)-C₁-C₆-alkyl, (C₁-C₆-alkoxycarbonyl)-C₁-C₆-alkyl, (hydroxycarbonyl)-C₂-C₆-alkenyl, (C₁-C₆-alkoxycarbonyl)-C₂-C₆-alkenyl, (hydroxycarbonyl)-C₁-C₄-alkoxy, (C₁-C₄-alkoxycarbonyl)-C₁-C₄-alkoxy, (C₁-C₄-alkylcarbonyl)oxy-C₁-C₄-alkyl, hydroxycarbonyl-C₁-C₄-alkoxy-C₁-C₄-alkyl, (C₁-C₄-alkylsulfonyl)oxy-C₁-C₄-alkyl, C₁-C₄-alkyl-O-C(O)-[C₁-C₄-alkyl-O]₃-C₁-C₄-alkyl, carbamoyloxy-C₁-C₄-alkyl, (C₁-C₄-alkylaminocarbonyl) oxy-C₁-C₄-alkyl, [di(C₁-C₄-alkyl)aminocarbonyl]oxy-C₁-C₄-alkyl, [(C₁-C₄-haloalkylsulfonyl)aminocarbonyl]oxy-C₁-C₄-alkyl, benzyloxy, where the phenyl ring may be substituted by 1 to 3 radicals from the group consisting of halogen and C₁-C₄-alkyl, amino, C₁-C₄-alkylamino, di(C₁-C₄-alkyl)amino, (C₁-C₄-alkylsulfonyl)amino, C₁-C₄-(haloalkylsulfonyl)amino, (C₁-C₄-alkylcarbonyl)amino, carbamoylamino, (C₁-C₄-alkylamino)carbonylamino, [di (C₁-C₄-alkyl)amino]carbonylamino, [(C₁-C₄-haloalkylsulfonyl)aminocarbonyl]amino, phenyl or heterocyclyl, where the phenyl and the heterocyclyl radical of the two last-mentioned substituents may carry one to three radicals from the following group: halogen, nitro, C₁-C₄-alkyl, C₁-C₄-haloalkyl, hydroxycarbonyl and C₁-C₆-alkoxycarbonyl;
R¹³, R¹⁴, R¹⁵ are hydrogen, halogen, cyano, C₁-C₆-alkyl, C₁-C₆-haloalkyl, C₁-C₆alkoxy, C₁-C₆-haloalkoxy, nitro, hydroxyl, C₁-C₄-alkylthio or benzyloxy;
R¹⁶ is hydrogen, C₁-C₆-alkyl, tri(C₁-C₆-alkyl)silyl, C₃-C₆-cycloalkyl, C₃-C₆-alkenyl, C₃-C₆-alkynyl, C₃-C₆-haloalkenyl, C₃-C₆-haloalkynyl, formyl, C₁-C₆-alkylcarbonyl, C₃-C₆-cycloalkylcarbonyl, C₂-C₆-alkenylcarbonyl, C₂-C₆-alkynylcarbonyl, C₁-C₆-alkoxycarbonyl, C₃-C₆-alkenyloxycarbonyl, C₃-C₆-alkynyloxycarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, C₁-C₆-haloalkylsulfonylaminocarbonyl, di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, di(C₁-C₆-alkyl)aminothiocarbonyl, C₁-C₆-alkylcarbonyl-C₁-C₆-alkyl, C₁-C₆-alkoxyimino-C₁-C₆-alkyl, N-(C₁-C₆-alkylamino)imino-C₁-C₆-alkyl or N-(di-C₁-C₆-alkylamino)imino-C₁-C₆-alkyl,
where the alkyl, cycloalkyl and alkoxy radicals mentioned may be partially or fully halogenated and/or may carry one to three of the following groups: cyano, hydroxyl, C₃-C₆-cycloalkyl, C₁-C₄-alkoxy, C₁-C₄-alkylthio, di(C₁-C₄-alkyl) amino, C₁-C₄-alkylcarbonyl, hydroxycarbonyl, C₁-C₄-alkoxycarbonyl, aminocarbonyl, C₁-C₄-alkylaminocarbonyl, di(C₁-C₄-alkyl)-aminocarbonyl or C₁-C₄-alkylcarbonyloxy;
phenyl, phenyl-C₁-C₆-alkyl, phenylcarbonyl, phenylcarbonyl- C₁-C₆-alkyl, phenoxycarbonyl, phenylaminocarbonyl, phenylsulfonylaminocarbonyl, N-(C₁-C₆-alkyl)-N-(phenyl)aminocarbonyl, phenyl-C₁-C₆-alkylcarbonyl, heterocyclyl, heterocyclyl-C₁-C₆-alkyl, heterocyclylcarbonyl, heterocyclylcarbonyl-C₁-C₆-alkyl, heterocyclyloxycarbonyl, heterocyclylaminocarbonyl, heterocyclylsulfonylaminocarbonyl, N-(C₁-C₆-alkyl)-N-(heterocyclyl)aminocarbonyl or heterocyclyl-C₁-C₆-alkylcarbonyl,
where the phenyl and the heterocyclyl radical of the 17 last-mentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy
SO₂R¹⁷; -C(O)-[C₁-C₄-alkyl-O]₃-C₁-C₄alkyl; or -C(O)-O-C₁-C₄-alkyl-O-phenyl, where the phenyl radical may optionally be substituted by one to three radicals from the group consisting of halogen and C₁-C₄-alkyl;
R¹⁷ is C₁-C₆-alkyl, C₁-C₆-haloalkyl or phenyl, where the phenyl radical may be partially or fully halogenated and/or may carry one to three of the following groups: C₁-C₆-alkyl, C₁-C₆-haloalkyl or C₁-C₆-alkoxy;
or an agriculturally useful salt thereof.

2. The benzoyl-substituted phenylalanineamide of the formula I according to claim 1, where R¹ is halogen or C₁-C₆-haloalkyl.

3. The benzoyl-substituted phenylalanineamide of the formula according to claim 1 or 2, where R² and R³ independently of one another are hydrogen, halogen or C₁-C₆-haloalkyl.

4. The benzoyl-substituted phenylalanineamide of the formula I according to any of claims 1 to 3, where R⁴, R⁵, R⁶, R⁷, R¹⁰, R¹³, R¹⁴ and R¹⁵ are hydrogen.

5. A process for preparing benzoyl-substituted phenylalanineamides of the formula I according to claim 1, which comprises
reacting phenylalanines of the formula V 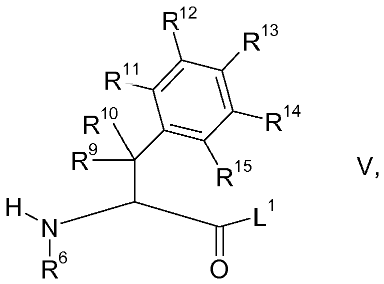 where R⁶ and R⁹ to R¹⁵ are as defined in claim 1 and L¹ is a nucleophilically displaceable leaving group,
with benzoic acids or benzoic acid derivatives of the formula IV 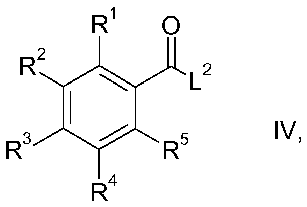 where R¹ to R⁵ are as defined in claim 1 and L² is a nucleophilically displaceable leaving group
to give corresponding benzoyl derivatives of the formula III 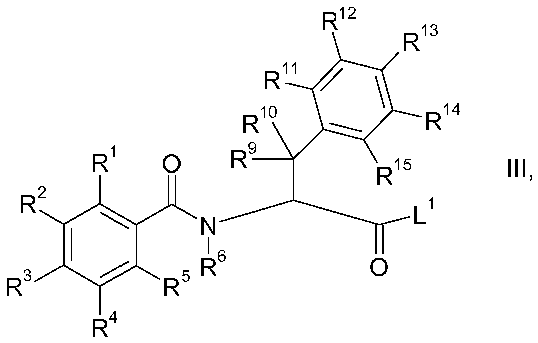 where R¹ to R⁶ and R⁹ to R¹⁵ are as defined in claim 1 and L¹ is a nucleophilically displaceable leaving group
and then reacting the resulting benzoyl derivatives of the formula III with an amine of the formula II
HNR⁷R⁸ II,
where R⁷ and R⁸ are as defined in claim 1.

6. A process for preparing benzoyl-substituted phenylalanineamides of the formula I according to claim 1, where R⁹ is hydroxyl and R¹⁰ is hydrogen, which comprises preparing benzoyl derivatives of the formula III where R⁹ is hydroxyl and R¹⁰ is hydrogen by acylation of keto compounds of the formula XIII 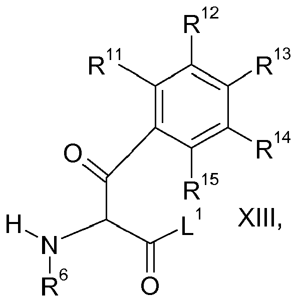 where R⁶ and R¹¹ to R¹⁵ are as defined in claim 1 and L¹ is a nucleophilically displaceable leaving group
with benzoic acids/benzoic acid derivatives of the formula IV into N-acyl keto compounds of the formula XII 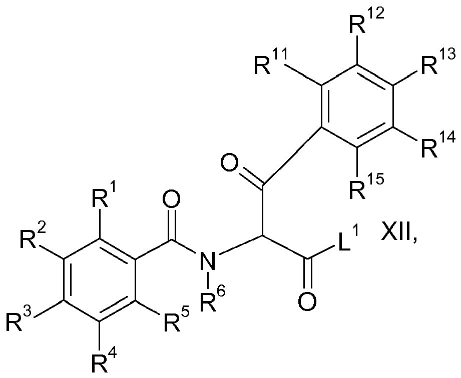 where R¹ to R⁶ and R¹¹ to R¹⁵ are as defined in claim 1 and L¹ is a nucleophilically displaceable leaving group, followed by reduction of the keto group.

7. A benzoyl derivative of the formula III 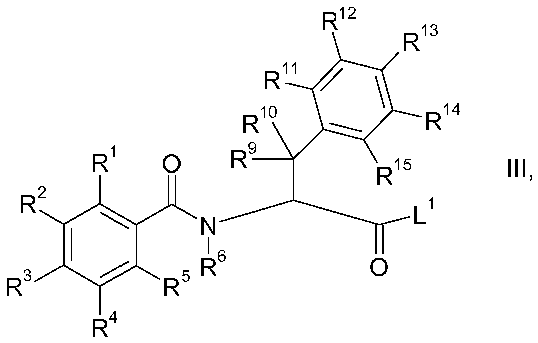 where R¹ to R⁶ and R⁹ to R¹⁵ are as defined in claim 1 and L¹ is a nucleophilically displaceable leaving group.

8. A composition, comprising a herbicidally effective amount of at least one benzoyl-substituted phenylalanineamide of the formula I or an agriculturally useful salt of I according to any of claims 1 to 4 and auxiliaries customary for formulating crop protection agents.

9. A process for preparing compositions according to claim 8, which comprises mixing a herbicidally effective amount of at least one benzoyl-substituted phenylalanineamide of the formula I or an agriculturally useful salt of I according to any of claims 1 to 4 and auxiliaries customary for formulating crop protection agents.

10. A method for controlling unwanted vegetation, which comprises allowing a herbicidally effective amount of at least one benzoyl-substituted phenylalanineamide of the formula I or an agriculturally useful salt of I according to any of claims 1 to 4 to act on plants, their habitat and/or on seed.

11. The use of a benzoyl-substituted phenylalanineamide of the formula I according to any of claims 1 to 4 or an agriculturally useful salt thereof as a herbicide.

## Revendications

1. Phénylalanine-amides substitués par benzoyle, de formule I 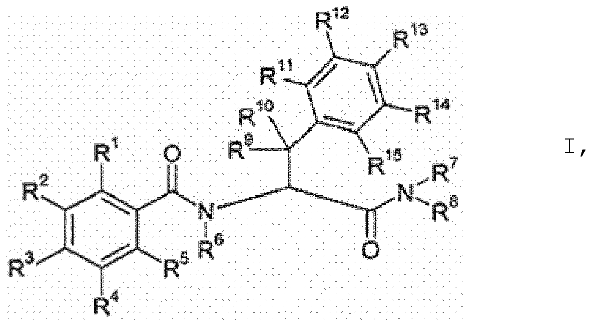 dans laquelle les variables ont les significations suivantes :
R¹ représente un atome d'halogène, un groupe cyano, alkyle en C₁-C₆, halogénoalkyle (C₁-C₆), halogénoalcoxy(C₁-C₆), nitro, hydroxycarbonyle, alcoxy(C₁-C₆)carbonyle, halogénoalkyl(C₁-C₆)thio ou phényle ;
R², R³, R⁴, R⁵ représentent un atome d' hydrogène ou d'halogène, un groupe cyano, alkyle en C₁-C₆, halogénoalkyle (C₁-C₆), alcoxy en C₁-C₆, halogénoalcoxy(C₁-C₆), nitro, amino, alkyl(C₁-C₆) amino, di[alkyl(C₁-C₆)] amino, alkyl(C₁-C₆)thio ou alcoxy(C₁-C₆)-carbonyle ;
R⁶, R⁷ représentent un atome d'hydrogène, un groupe hydroxy ou alcoxy en C₁-C₆;
R⁸ représente un groupe alkyle en C₁-C₆, cyanoalkyle (C₁-C₄) ou halogénoalkyle(C₁-C₆) ;
R⁹ représente OR¹⁶
R¹⁰ représente un atome d'hydrogène ou un groupe alkyle en C₁-C₆;
R¹¹, R¹² représentent un atome d'hydrogène ou d'halogène, un groupe cyano, alkyle en C₁-C₆, halogénoalkyle(C₁-C₆), hydroxy, alcoxy en C₁-C₆, halogénoalcoxy(C₁-C₆), hydroxy, nitro, hydroxy-alkyle(C₁-C₄) , alcoxy(C₁-C₆)-alkyle(C₁-C₄), tri[alkyl(C₁-C₆)]silyloxy-alkyle(C₁-C₄), alkyl(C₁-C₄)thio, (hydroxycarbonyl)-alkyle(C₁-C₆), [alcoxy (C₁-C₆)carbonyl]-alkyle(C₁-C₆), (hydroxycarbonyl)-alcényle(C₂-C₆), [alcoxy (C₁-C₆) carbonyl]-alcényle(C₂-C₆), (hydroxycarbonyl)-alcoxy(C₁-C₄), (alcoxy (C₁-C₄)carbonyl)-alcoxy(C₁-C₄), [alkyl(C₁-C₄)carbonyl]oxy-alkyle(C₁-C₄), hydroxycarbonyl-alcoxy(C₁-C₄)-alkyle(C₁-C₄), [alkyl(C₁-C₄)sulfonyl]oxy-alkyle(C₁-C₄), alkyl(C₁-C₄)-O-C(O)-[alkyl(C₁-C₄)-O]₃-alkyle(C₁-C₄), carbamoyloxy-alkyle(C₁-C₄), [alkyl(C₁-C₄)aminocarbonyl]oxy-alkyle(C₁-C₄), [di(alkyl(C₁-C₄))aminocarbonyl]oxy-alkyle(C₁-C₄), [(halogénoalkyl (C₁-C₄) sulfonyl)-aminocarbonyl]oxy-alkyle(C₁-C₄) benzyloxy, le cycle phényle pouvant être substitué par 1 à 3 radicaux choisi dans le groupe constitué par halogéno et alkyle en C₁-C₄, amino, alkyl(C₁-C₄)amino, di[alkyl(C₁-C₄)]amino, [alkyl(C₁-C₄)sulfonyl]amino, [halogénoalkyl(C₁-C₄)sulfonyl]amino, [alkyl(C₁-C₄)carbonyl]amino, carbamoylamino, [alkyl(C₁-C₄)amino]carbonylamino, [di(alkyl(C₁-C₄))amino]-carbonylamino, [(halogénoalkyl(C₁-C₄)sulfonyl)aminocarbonyl]-amino, phényle ou hétérocyclyle, le radical phényle et le radical hétérocyclyle des deux substituants nommés en dernier pouvant porter un à trois radicaux choisis dans le groupe suivant : halogéno, nitro, alkyle en C₁-C₄, halogénoalkyle(C₁-C₄), hydroxycarbonyle et alcoxy(C₁-C₆)carbonyle ;
R¹³, R¹⁴, R¹⁵ représentent un atome d'hydrogène, un atome d'halogène, un groupe cyano, alkyle en C₁-C₆, halogénoalkyle(C₁-C₆), alcoxy en C₁-C₆, halogénoalcoxy(C₁-C₆), nitro, hydroxy, alkyl(C₁-C₄)thio ou benzyloxy ;
R¹⁶ représente un atome d'hydrogène, un groupe alkyle en C₁-C₆, tri[alkyl(C₁-C₆)]silyle cycloalkyle en C₃-C₆, alcényle en C₃-C₆, alcynyle en C₃-C₆, halogénoalcényle(C₃-C₆), halogénoalcynyle(C₃-C₆), formyle, alkyl(C₁-C₆)carbonyle, cycloalkyl(C₃-C₆)-carbonyle, alcényl(C₂-C₆)carbonyle, alcynyl(C₂-C₆)carbonyle, alcoxy (C₁-C₆) carbonyle, alcényloxy(C₃-C₆) carbonyle, alcynyloxy(C₃-C₆)-carbonyle, alkyl(C₁-C₆)aminocarbonyle, alcényl(C₃-C₆)aminocarbonyle, alcynyl(C₃-C₆) aminocarbonyle, alkyl(C₁-C₆)sulfonylaminocarbonyle, halogénoalkyl(C₁-C₆)sulfonylaminocarbonyle, di[alkyl(C₁-C₆)]-aminocarbonyle, N-[alcényl(C₃-C₆)]- N-[alkyl(C₁-C₆)]-aminocarbonyle, N-[alcynyl(C₃-C₆)]-N-[alkyl(C₁-C₆)]-aminocarbonyle, N-[alcoxy(C₁-C₆)]-N-[alkyl(C₁-C₆)]-aminocarbonyle, N-[alcényl(C₃-C₆)]-N-[alcoxy(C₁-C₆)]-aminocarbonyle, N-[alcynyl(C₃-C₆)]-N-[alcoxy(C₁-C₆)]-aminocarbonyle, di[alkyl(C₁-C₆)]-aminothiocarbonyle, alkyl(C₁-C₆)carbonyl-alkyle(C₁-C₆), alcoxy(C₁-C₆)-imino-alkyle (C₁-C₆), N-[alkyl(C₁-C₆)amino]-imino-alkyle(C₁-C₆) ou N-[dialkyl(C₁-C₆)amino]-imino-alkyle (C₁-C₆),
les radicaux alkyle, cycloalkyle et alcoxy nommés pouvant être partiellement ou totalement halogénés et/ou pouvant porter un à trois des groupes suivants : cyano, hydroxy, cycloalkyle en C₃-C₆, alcoxy en C₁-C₄, alkyl(C₁-C₄)thio, di[alkyl(C₁-C₄)]amino, alkyl(C₁-C₄)carbonyle, hydroxycarbonyle, alcoxy(C₁-C₄)carbonyle, aminocarbonyle, alkyl(C₁-C₄)aminocarbonyle, di[alkyl(C₁-C₄)]-aminocarbonyle ou alkyl(C₁-C₄)carbonyloxy ;
phényle, phényl-alkyle(C₁-C₆), phénylcarbonyle, phénylcarbonyl-alkyle(C₁-C₆), phénoxycarbonyle, phénylaminocarbonyle, phénylsulfonylamino-carbonyle, N-[alkyl(C₁-C₆)]-N-(phényl)-amino-carbonyle, phényl-alkyl(C₁-C₆)carbonyle, hétérocyclyle, hétérocyclyl-alkyle(C₁-C₆), hétérocyclylcarbonyle, hétérocyclylcarbonyl-alkyle(C₁-C₆), hétérocyclyloxycarbonyle, hétérocyclylaminocarbonyle, hétérocyclylsulfonyl-aminocarbonyle, N-[alkyl(C₁-C₆)]-N-(hétérocyclyl)- aminocarbonyle, ou hétérocyclyl-alkyl(C₁-C₆)carbonyle,
le radical phényle et le radical hétérocyclyle des 17 substituants nommés en dernier pouvant être partiellement ou totalement halogénés et/ou pouvant porter un à trois des groupes suivants : nitro, cyano, alkyle en C₁-C₄, halogénoalkyle(C₁-C₄), alcoxy en C₁-C₄ ou halogénoalcoxy(C₁-C₄)
SO₂R¹⁷; -C(O)-[alkyl(C₁-C₄)-O]₃-alkyle(C₁-C₄); ou -C(O)-O-alkyl(C₁-C₄)-O-phényle, le radical phényle pouvant éventuellement être substitué par un à trois radicaux choisis dans le groupe constitué par halogéno et alkyle en C₁-C₄ ;
R¹⁷ représente un groupe alkyle en C₁-C₆, halogénoalkyle(C₁-C₆) ou phényle,
le radical phényle pouvant être partiellement ou totalement halogéné et/ou pouvant porter un à trois des groupes suivants : alkyle en C₁-C₆, halogénoalkyle(C₁-C₆) ou alcoxy en C₁-C₆ ;
ainsi que leurs sels utilisables en agriculture.

2. Phénylalanine-amides substitués par benzoyle de formule I selon la revendication 1, dans lesquels R¹ représente un atome d'halogène ou un groupe halogénoalkyle en C₁-C₆.

3. Phénylalanine-amides substitués par benzoyle de formule I selon la revendication 1 ou 2, dans lesquels R² et R³ représentent indépendamment l'un de l'autre un atome d'hydrogène ou d'halogène, ou un groupe halogénoalkyle en C₁-C₆.

4. Phénylalanine-amides substitués par benzoyle de formule I selon les revendications 1 à 3, dans lesquels R⁴, R⁵, R⁶, R⁷, R¹⁰, R¹³, R¹⁴ et R¹⁵ représentent des atomes d'hydrogène.

5. Procédé pour la préparation de phénylalanine-amides substitués par benzoyle, de formule I selon la revendication 1, **caractérisé en ce**
**qu'**on fait réagir des phénylalanines de formule V 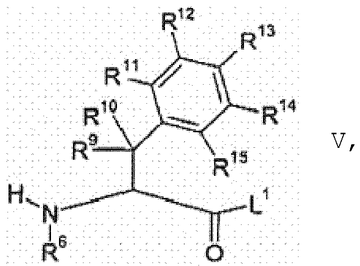 dans laquelle R⁶ et R⁹ à R¹⁵ ont les significations données dans la revendication 1 et L¹ représente un groupe partant pouvant être remplacé par substitution nucléophile,
avec des acides benzoïques ou dérivés d'acides benzoïques de formule IV 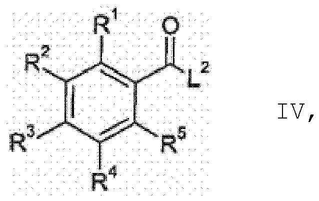 dans laquelle R¹ à R⁵ ont les significations données dans la revendication 1 et L² représente un groupe partant pouvant être remplacé par substitution nucléophile,
pour aboutir aux dérivés benzoyle correspondants de formule III 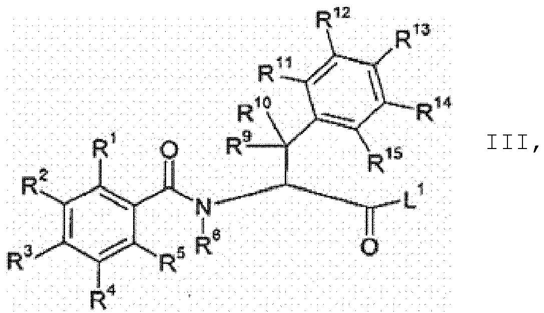 dans laquelle R¹ à R⁶ et R⁹ à R¹⁵ ont les significations données dans la revendication 1 et L¹ représente un groupe partant pouvant être remplacé par substitution nucléophile,
et on fait ensuite réagir les dérivés benzoyle de formule III obtenus avec une amine de formule II
HNR⁷R⁸ II,
où R⁷ et R⁸ ont les significations données dans la revendication 1.

6. Procédé pour la préparation de phénylalanine-amides substitués par benzoyle, de formule I selon la revendication 1, dans lesquels R⁹ représente le groupe hydroxy et R¹⁰ représente un atome d'hydrogène, **caractérisé en ce qu'**on prépare des dérivés benzoyle de formule III dans lesquels R⁹ représente le groupe hydroxy et R¹⁰ représente un atome d'hydrogène, par acylation de composés céto de formule XIII 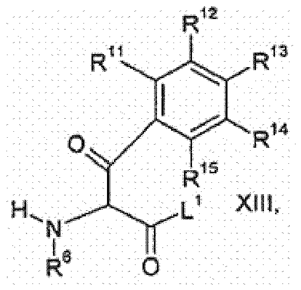 dans laquelle R⁶ ainsi que R¹¹ à R¹⁶ ont les significations données dans la revendication 1 et L¹ représente un groupe partant pouvant être séparé par substitution nucléophile,
avec des (dérivés d')acides benzoïques de formule IV, pour aboutir à des composés N-acyl-céto de formule XII 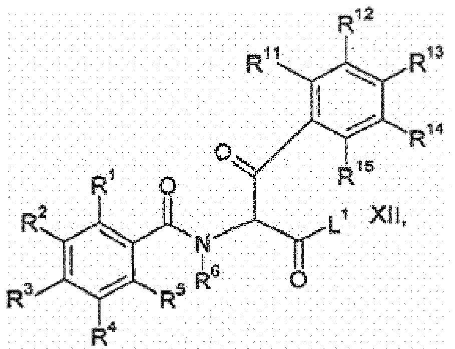 dans laquelle R¹ à R⁶ ainsi que R¹¹ à R¹⁵ ont les significations données dans la revendication 1 et L¹ représente un groupe partant séparable par substitution nucléophile, et réduction subséquente du groupe céto.

7. Dérivés benzoyle de formule III 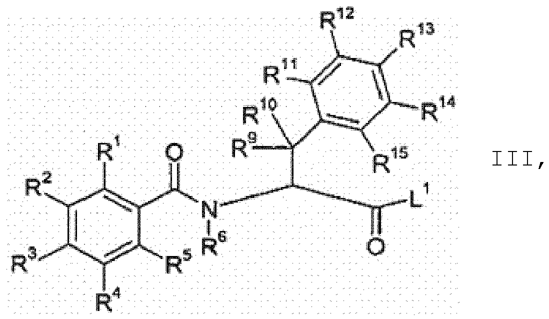 dans laquelle R¹ à R⁶ et R⁹ à R¹⁵ ont les significations données dans la revendication 1 et L¹ représente un groupe partant pouvant être remplacé par substitution nucléophile.

8. Compositions, contenant une quantité à effet herbicide d'au moins un phénylalanine-amide substitué par benzoyle, de formule I, ou d'un sel de I utilisable en agriculture, selon les revendications 1 à 4, et des adjuvants usuels pour la formulation de produits phytosanitaires.

9. Procédé pour la préparation de compositions selon la revendication 8, **caractérisé en ce qu'**on mélange une quantité à effet herbicide d'au moins un phénylalanine-amide substitué par benzoyle, de formule I, ou d'un sel de I utilisable en agriculture, selon les revendications 1 à 4, et des adjuvants usuels pour la formulation de produits phytosanitaires.

10. Procédé pour la lutte contre une croissance indésirable de plantes, **caractérisé en ce qu'**on fait agir sur des plantes, leur habitat et/ou sur des semences une quantité à effet herbicide d'au moins un phénylalanine-amide substitué par benzoyle, de formule I, ou d'un sel de I utilisable en agriculture, selon les revendications 1 à 4.

11. Utilisation des phénylalanine-amides substitués par benzoyle, de formule I, selon les revendications 1 à 4, et de leurs sels utilisables en agriculture, en tant qu'herbicides.
